# EUROPEAN PATENT SPECIFICATION

(11) **EP 0 445 731 B1**
(45) Date of publication and mention of the grant of the patent: **10.01.1996**
(21) Application number: 91103308.2
(22) Date of filing: 05.03.1991
(51) Int. Cl.: C07D 487/08, C07D 453/02, C07D 451/04, C07D 211/72, A61K 31/40, A61K 31/435, A61K 31/445, A61K 31/46

(54) **Azabicyclo and azacyclo oxime and amine cholinergic agents and pharmaceutically acceptable salts thereof**
Azabicyclo- und Azacyclooxime und -amine mit cholinergischer Wirkung und ihre pharmazeutisch annehmbaren Salze
Oximes et amines azabicycliques et azacycliques douées d'une activité cholinergique et leurs sels pharmaceutiquement acceptables

(30) Priority: 06.03.1990 US 488916; 02.10.1990 US 591647; 22.01.1991 US 641478
(43) Date of publication of application: 11.09.1991
(73) Proprietor: WARNER-LAMBERT COMPANY, Morris Plains New Jersey 07950 (US)
(72) Inventor: Lauffer, David Jeffrey, Saline, Michigan 48176 (US); Moos, Walter Hamilton, Ann Arbor, Michigan 48105 (US); Pavia, Michael Raymond, Ann Arbor, Michigan 48103 (US); Tecle, Haile, Ypsilanti, Michigan 48197 (US); Thomas, Anthony Jerome, Ann Arbor, Michigan 48104 (US)
(74) Representative: Mansmann, Ivo

(56) References cited:
- DE-A- 2 019 536
- DE-A- 2 552 176
- US-A- 4 353 922

## Description

### FIELD OF INVENTION

The present invention is a class of oximes and amines which are muscarinic agonists, rendering them useful as pharmaceutical agents. More specifically, the compounds are azabicyclic and azacyclic oximes and amines.

### BACKGROUND

Disorders of cognition are generally characterized by symptoms of forgetfulness, confusion, memory loss, attentional deficits, and/or, in some cases, affective disturbances. These symptoms may arise as a result of the general aging process and/or from organic brain disease, cerebrovascular disease, head injury, or developmental or genetic defects.

The general decrease in cognitive function which accompanies the aging process is well accepted. The same phenomenon has been observed and documented in many lower mammals, including those routinely employed in pharmacological testing programs for screening and predicting usefulness for particular drugs in higher animals, including humans.

Although disorders of cognition often accompany the general aging process, presenile and senile primary degenerative dementia are the most common accepted causes of mental deterioration in the elderly. It has been estimated that at least ten percent of persons over 60 years of age will eventually suffer severe mental deterioration. A much larger number will experience cognitive decline of sufficient severity to impede their activities.

Many of the symptoms of cognitive disorders, especially impaired memory, are associated with decreased acetylcholine synthesis and the impairment of cholinoreceptive neurons. In the hippocampus and cerebral cortex of patients suffering from primary degenerative dementia, for example, the level of the enzyme choline acetyltransferase (CAT) can be reduced by as much as 90%. (See Davies, et al, The Lancet, 1976 (Vol. 2):1403; Perry, et al, J. Neurol. Sci., 34:247-265 (1977); and White, et al, The Lancet, 1977 (Vol. 1):668-670).

Since CAT catalyzes the synthesis of acetylcholine from its precursors choline and acetyl coenzyme A, the loss of CAT reflects the loss of cholinergic, or acetylcholine-releasing, nerve endings in the hippocampus and cerebral cortex. There is abundant evidence that cholinergic terminals in the hippocampus are critically important for memory formation.

The cholinergic hypothesis suggests that drugs which restore acetylcholine levels or which mimic the action of acetylcholine (i.e., are cholinomimetic) are effective in correcting this deficit in neurotransmitter chemical and provide treatment of the memory impairment symptom of cerebral insufficiency. Considerable biochemical, pharmacological, and electrophysiological evidence supports the hypothesis that deficits in the cholinergic system underlie geriatric cognitive dysfunction. (See C. Peterson and G. E. Gibson, Neurobiol. Aging, 4:25-30 (1983)). Aged humans and nonhuman primates with decreased cognition show improved memory when they are treated, for example, with acetylcholinesterase inhibitors such as physostigmine. These agents increase the available supply of synaptic acetylcholine by inhibiting its hydrolysis.

Aminopyridines such as 3,4-diaminopyridine ameliorate age-related cognitive deficits by increasing the release of acetylcholine from presynaptic nerve terminals, thus increasing synaptic acetylcholine. (See H. P. Davis, et al, Exp. Aging Res., 9:211-214 (1983)).

It has been known for some time that the natural alkaloid, muscarine, has the ability to act relatively selectively at autonomic effector cells to produce qualitatively the same effects as acetylcholine. Two related alkaloids, pilocarpine and arecoline (the methyl ester of 1,2,5,6-tetrahydro-1-methyl-3-pyridinecarboxylic acid), have the same principal sites of action as muscarine and acetylcholine and are thus classified as having "muscarinic" action. Although these naturally occurring alkaloids are of great value as pharmacological tools, present clinical use is largely restricted to the use of pilocarpine as miotic agent.

Recently it has been demonstrated that arecoline is effectively in ameliorating some of the symptoms of cognitive disorders in patients clinically diagnosed as having presenile primary degenerative dementia. Significant improvement was observed in a test of picture recognition after administration of arecoline to patients in a double-blind study. (See Christie, et al, Brit. J. Psychiatry, 138:46-50 (1981)).

### INFORMATION DISCLOSURE STATEMENT

Certain 3- or 4-ketoximes of 1-(lower alkyl)-1,2,5,6-tetrahydropyridines in which the oxygen is unsubstituted are disclosed in United States Patent 3,004,979, having utility as parasympathomimetic agents acting on nonstriated muscle.

U.S. Patent 4,710,508 describes O-substituted 1-(1,2,3,6-tetrahydro-1-methyl-3-pyridinyl)ketone oximes and O-substituted 1-(1,2,3,6-tetrahydro-4-pyridinyl)ketone oximes which are useful as analgesic agents or agents for the treatment of the symptoms of cerebral insufficiency characterized by decreased central acetylcholine production.

Dissertation Abstracts Int. B 1984, 45(7), 2120; CA102:113440 m describes oxime-O-ethers of the following formula as having anticholinergic properties: 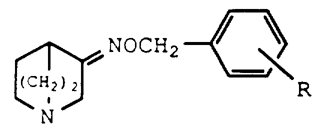 wherein R is F, Cl, Br, NO₂, OCH₃, CF₃, or CH₃. Particularly relevant are pages 128-136, 166, 167, 198-203.

French Patent 2,086,292 describes 3-quinuclidinone oxime carbamates of the following formula having insecticidal and acaricidal activity. 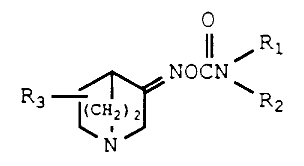 wherein R₃ is alkyl, alkenyl, alkylidene, halogen, cyano, haloalkyl, haloalkenyl, alkoxy, etc; R₁ and R₂ are hydrogen, lower alkyl, acyl which may be substituted, lower alkenyl, or the group R-O-CH₂- wherein R is methyl, ethyl, isopropyl; propyl-1,2-diene, allyl, 1-methyl-2-propenyl; phenyl or benzyl.

### SUMMARY OF THE INVENTION

The present invention is directed to compounds of the following general Formulas I, II, III, and IV and pharmaceutically acceptable salts thereof. 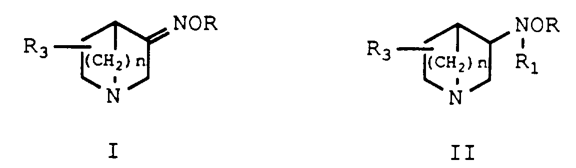 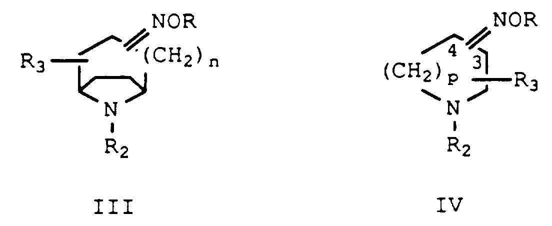 wherein n is one or two; wherein p is zero or one to four, wherein each of R₁ and R₂ is hydrogen or straight or branched lower alkyl having from 1 to 4 carbon atoms; wherein R₃ is hydrogen, a straight or branched lower alkyl group having from 1 to 6 carbon atoms, or cycloalkyl having from 3 to 6 carbon atoms, hydroxy, a straight or branched lower alkoxy group having from 1 to 4 carbon atoms, an acyloxy group wherein the acyl moiety has from 2 to 5 carbon atoms or the group -(CH₂) _{q}NR₈R₉ wherein q is zero or one to four and each of R₈ and R₉ is the same or different and is hydrogen or a straight or branched lower alkyl group having from 1 to 4 carbon atoms; wherein R is hydrogen or the group:

-Y-Z

wherein Y is a bond or a straight or branched hydrocarbon chain having from 1 to 20 carbon atoms and optionally contains from 1 to 4 double and/or triple bonds; and Z is hydrogen;
a cycloalkyl group having from 3 to 6 carbon atoms;
an aromatic group selected from phenyl, 2- or 3-thienyl, 2- or 3-furanyl, 2- or 3-pyrrolyl wherein the aromatic group is unsubstituted or is substituted with 1 or 2 substituents selected from chlorine, bromine, fluorine, trifluoromethyl, nitro, amino, amino substituted with 1 or 2 straight or branched lower alkyl groups having from 1 to 4 carbon atoms, or straight or branched alkoxy having from 1 to 4 carbon atoms;
-NR₄R₅ wherein R₄ and R₅ are the same or different and are hydrogen or straight or branched alkyl having from 1 to 4 carbon atoms; 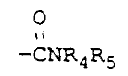
wherein R₄ and R₅ have the meanings defined above; 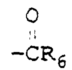
wherein R₆ is a straight or branched alkyl group having from 1 to 6 carbon atoms;
-CN;
-CO₂R₇ wherein R₇ is hydrogen, a straight or branched lower alkyl having from 1 to 6 carbon atoms and is straight or branched, or benzyl; or
-XR₁₀ wherein X is oxygen or sulfur and R₁₀ is a straight or branched hydrocarbon chain having from 1 to 6 carbon atoms and which optionally contains 1 or 2 double or triple bonds;
with the proviso that the following compounds are excluded: (a) compounds of Formula I wherein n is two, and R is hydrogen, or the group Y-Z wherein Y is a bond and Z is -C(=O)NR₄R₅ or -C(=O)R₆; and (b) compounds of Formulas III and IV wherein R is hydrogen.

Pharmaceutical compositions comprising compounds of Formulas I, II, III, or IV are also included in the present invention as well as methods of using the compounds for the preparation of pharmaceutical compositions useful as analgesics and in the treatment of senile cognitive decline.

### DETAILED DESCRIPTION OF THE INVENTION

In the compounds depicted by general Formulas I, II, III, and IV the various substituents are further described as follows. Illustrative examples of straight or branched lower alkyl having from 1 to 4 carbon atoms or from 1 to 6 carbon atoms included methyl, ethyl, n-propyl, isopropyl, n-butyl, tert-butyl, n-pentyl, n-hexyl.

Illustrative of lower alkoxy groups having from 1 to 4 carbon atoms are methoxy, ethoxy, and n-propoxy.

Illustrative examples of a saturated straight or branched hydrocarbon chain having from 1 to 8 carbon atoms include n-octyl, n-heptyl, and all the illustrative examples of straight or branched lower alkyl groups having from 1 to 6 carbon atoms set forth above.

Illustrative examples of straight or branched unsaturated hydrocarbon chains which contain from 1 to 3 unsaturations which are double or triple bonds are ethenyl, 2,4-pentadienyl, 1,4-pentadienyl, 2,4-pentadiynyl, 1,4-pentadiynyl, (E&Z) 2-penten-4-ynyl, 2-pentyn-4-enyl, 2-propenyl, 3-butenyl, 1-methyl-2-propenyl, 2-pentenyl, 2-ethyl-3-butenyl, 4-hexenyl, ethynyl, 2-propynyl, 1-methyl-2-propynyl, 1-ethyl-2-propynyl, 1-methyl-3-butynyl, 3-butynyl, or 4-pentynyl. Illustrative examples of cycloalkyl groups having from 3 to 6 carbon atoms are cyclopropyl, cyclobutyl, and cyclohexyl.

Pharmaceutically acceptable acid addition salts of the compounds of Formulas I, II, III, and IV are illustratively hydrochloric, sulfuric, phosphoric, acetic, benzoic, citric, malonic, salicylic, malic, fumaric, oxalic, succinic, tartaric, lactic, gluconic, ascorbic, maleic, aspartic, benzenesulfonic, methane and ethanesulfonic, hydroxymethane- and hydroxyethanesulfonic. (See, for example, "Pharmaceutical Salts," J. Pharm. Sci. 66(1):1-19 (1977).

Preferred compounds of the present invention are those of Formulas I and III with the compounds of Formula I being more preferred. The most preferred compounds of this invention are those of Formula I wherein n is one, i.e., compounds of the following Formula V: 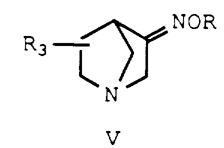 In general, Formula V R and R₃ have the same meanings as set forth above for Formulas I, II, III, and IV. Compounds of Formula V wherein R₃ is hydrogen and R is other than hydrogen or Y-Z wherein Z is NR₄R₅ are more preferred.

Compounds of Formula I wherein n is two may be depicted as set forth below in Formula VI: 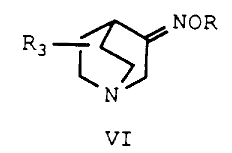 wherein R and R₃ have the meanings defined above.

Compounds of Formula II wherein n is one may be depicted as set forth below in Formula VII and wherein n is two may be depicted as set forth below in Formula VIII: 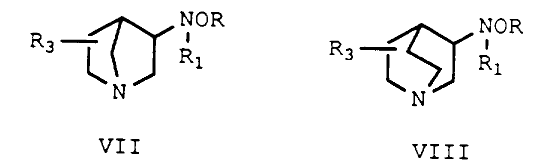 In Formulas VII and VIII the substituent groups represented by R₃, R, and R₁ have the meanings defined hereinabove in Formulas I, II, III, and IV.

In the compounds of Formulas III and IV the =NOR group is attached to either the 2-, 3-, or 4-position (Formula III) or the 3- or 4-position (Formula IV) of the ring.

In addition to the novel compounds of the present invention as represented by Formulas I through VIII above, the present invention provides novel pharmaceutical preparations.

The present invention provides pharmaceutical compositions useful as analgesic agents comprising an analgesically effective amount of a compound as defined above in combination with a pharmaceutically acceptable carrier. In another aspect, the present invention provides a method of alleviating pain in a mammal comprising administering to a mammal in need of such treatment an analgesically effective amount of a compound as defined above in combination with a pharmaceutically acceptable carrier.

In another aspect, the present invention provides pharmaceutical compositions for treating the symptoms of senile cognitive decline comprising a cholinergically effective amount of a compound as defined above in combination with a pharmaceutically acceptable carrier. In yet another aspect, the present invention provides a method of treating the symptoms of senile cognitive decline in the elderly characterized by decreased cerebral acetylcholine production or release comprising administering to a patient in need of such treatment of cholinergically effective amount of a compound as defined above in combination with a pharmaceutically acceptable carrier.

The compounds of the present invention may exist in either of two Z and E isomeric forms. The present invention includes both forms of the compounds as well as mixtures of the Z and E forms. Illustratively Formulas IX and X depict the Z and E forms of the compounds of Formula I. Moreover, in those compounds in which there is a double bond in a carbon chain, both the Z and E forms of the olefins are included in the present invention.

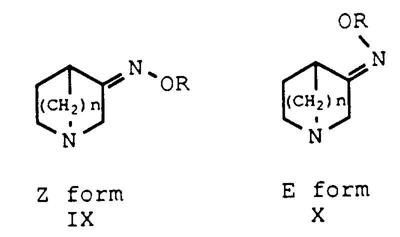

The compounds of Formulas I, III, and IV are prepared by reacting a ketone of the following Formulas XI, XII, and XIII with an amine of the formula NH₂OR˙ HCl wherein R and R₃ have the meanings defined in Formulas I, II, III, and IV as depicted below:

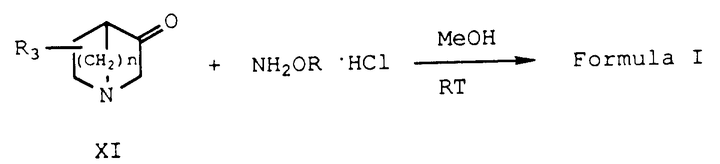

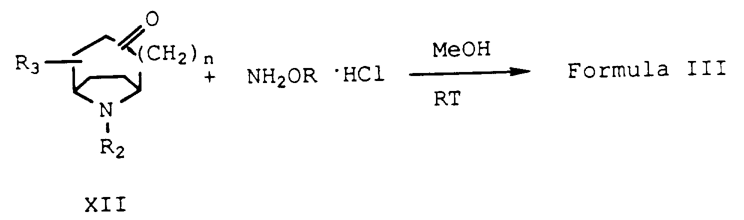

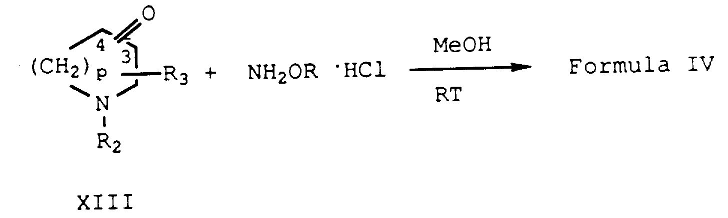

In each of the above depicted reactions, typically methanol is used as the solvent and the reaction is carried out at room temperature.

The compounds of Formula II are prepared by reducing Formula I compounds by procedures well known in the art, for example, by treatment with sodium cyanoborohydride under controlled pH conditions. Therefore, not only are the compounds of Formula I pharmaceutically useful they are also useful as starting material to make pharmaceutically useful compounds. Similarly, the compounds of Formula I wherein R is hydrogen may be used to prepare compounds of Formula I wherein R is the group -C(=O)R₆.

The salts are prepared by contacting the free base form of the compounds of this invention with a sufficient amount of the desired acid to produce the salt in the conventional manner. The free base forms may be regenerated, if desired, by treating the salt form with a base. For example, dilute aqueous solutions of such bases as sodium hydroxide, potassium carbonate, ammonia, and sodium bicarbonate maybe utilized for this purpose.

The free base forms of the compounds of this invention differ somewhat from their respective salt forms in such physical properties as melting point and solubility in polar solvents, but the salts are otherwise equivalent to their respective free acid or base forms for the purposes of the invention.

The starting materials represented by Formulas XI, XII, and XIII are known in the art or can be prepared by procedures generally known in the art. See, for example, J. Saunders, et al, J. Chem. Soc., Chem. Comm. 1988, 1618 and R. J. Snow and L. J. Street, Tet. Lett. 1989, 30, 5795. Also, the amines represented by NH₂OR are commercially available or may be prepared by procedures generally known in the art.

### EXAMPLE 1

### 1-Azabicyclo[2.2.1]hept-3-one oxime hydrochloride

1-Azabicyclo[2.2.1]hept-3-one was prepared by the procedure described in J. Chem. Soc., Chem. Comm. 1988, 1618.

1-Azabicyclo[2.2.1]hept-3-one (2 g, 18 mmol) and hydroxylamine hydrochloride (1.25 g, 18 mmol) was dissolved in 10 mL MeOH and stirred at room temperature for 18 hours. The reaction mixture was concentrated in vacuo. The crystalline residue was recrystallized from ethanol to afford 2.04 g (70%) of the title product, m.p. 211°C.

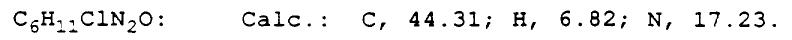

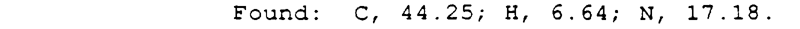

Mass Spec.: m/e 126.1 (M+ for free base)
¹H NMR: δ (CDCl₃) 0.87 (1H, m), 1.31-1.46 (1H, m), 2.39-2.57 (5H, m), 3.01-3.21 (2H, m), 10.15 (1H, s), 10.58-10.82 (1H, br s). ₁₃C NMR δ (CDCl₃) 25.2, 40, 50.5, 54.5, 58.5, 153.5

### EXAMPLE 2

### 1-Azabicyclo[2.2.1]hept-3-one, O-ethyloxime hydrochloride

1-Azabicyclo[2.2.1]hept-3-one (2 g, 18 mmol) was dissolved in 25 mL of methanol. Ethoxyamine hydrochloride (1.76 g, 18 mmol) was added and the reaction was stirred at room temperature for 18 ½ours. The reaction was concentrated in vacuo to afford a yellow solid. The residue was dissolved in water, basified with saturated aqueous potassium carbonate, and extracted with dichloromethane (3 x 150 mL). The combined extracts were dried over anhydrous sodium sulfate and concentrated in vacuo to afford a clear, yellow liquid. The liquid was dissolved in diethyl ether and filtered through Celite. The filtrate was treated with ethereal hydrogen chloride to yield 2.5 g (75%) of the title product, m.p. 158-160°C.

| | | | | |
|---|---|---|---|---|
| C₈H₁₅ClN₂O: | Calc.: | C, 50.39; | H, 7.93; | N, 14.69. |
| | Found: | C, 49.96; | H, 7.76; | N, 14.56. |

Mass Spec.: m/e 154.1 (M+ for free base)
¹H NMR: δ (CDCl₃) 1.20-1.28 (3H, t), 1.97-2.05 (1H, m), 240-253 (1H, m), 3.29-3.42 (2H, m), 3.52-3.62 (2H, m), 3.83-3.95 (2H, m), 4.06-4.14 (2H, q), 4.20-4.27 (1H, m). ¹³C NMR: δ (CDCl₃) 14.4, 26.1, 40, 51.7, 55.8, 59.6, 70.3, 153.1.

### EXAMPLE 3

### 1-Azabicyclo[2.2.1]heptan-3-one, O-methyloxime hydrochloride

1-Azabicyclo[2.2.1]hept-3-one (1.9 g, 17.1 mmol) was dissolved in 10 mL of methanol. Methoxyamine hydrochloride (1.4 g, 17.1 mmol) was added and the reaction was stirred at room temperature for 18 hours. The reaction was concentrated in vacuo to afford a white solid. Recrystallization of the residue from ethanol-diisopropyl ether afforded 2.27 g (75%) of the title product as a mixture of syn and anti isomers, m.p. 210-211°C.

| | | | | |
|---|---|---|---|---|
| C₇H₁₃ClN₂O: | Calc.: | C, 47.59; | H, 7.42; | N, 15.86. |
| | Found: | C, 47.46; | H, 7.54; | N, 15.84 |

Mass Spec.: m/e 140.1 (M+ for free base).
¹H NMR: δ (CDCl₃) 1.99 (1H, m), 2.36-2.52 (1H, m), 3.37-3.65 (4H, m), 3.86, 3.88 (3H, 2s) (3H, s), 3.89-4.00 (2H, m), 4.21-4.29 (1H, m). ₁₃C NMR: δ (CDCl₃) 25, 26, 37.2, 40, 51.5, 51.7, 55.7, 56.5, 59.0, 59.6, 62.3, 153, 153.5.

The above procedure was utilized for the synthesis of the products listed in Table 1.

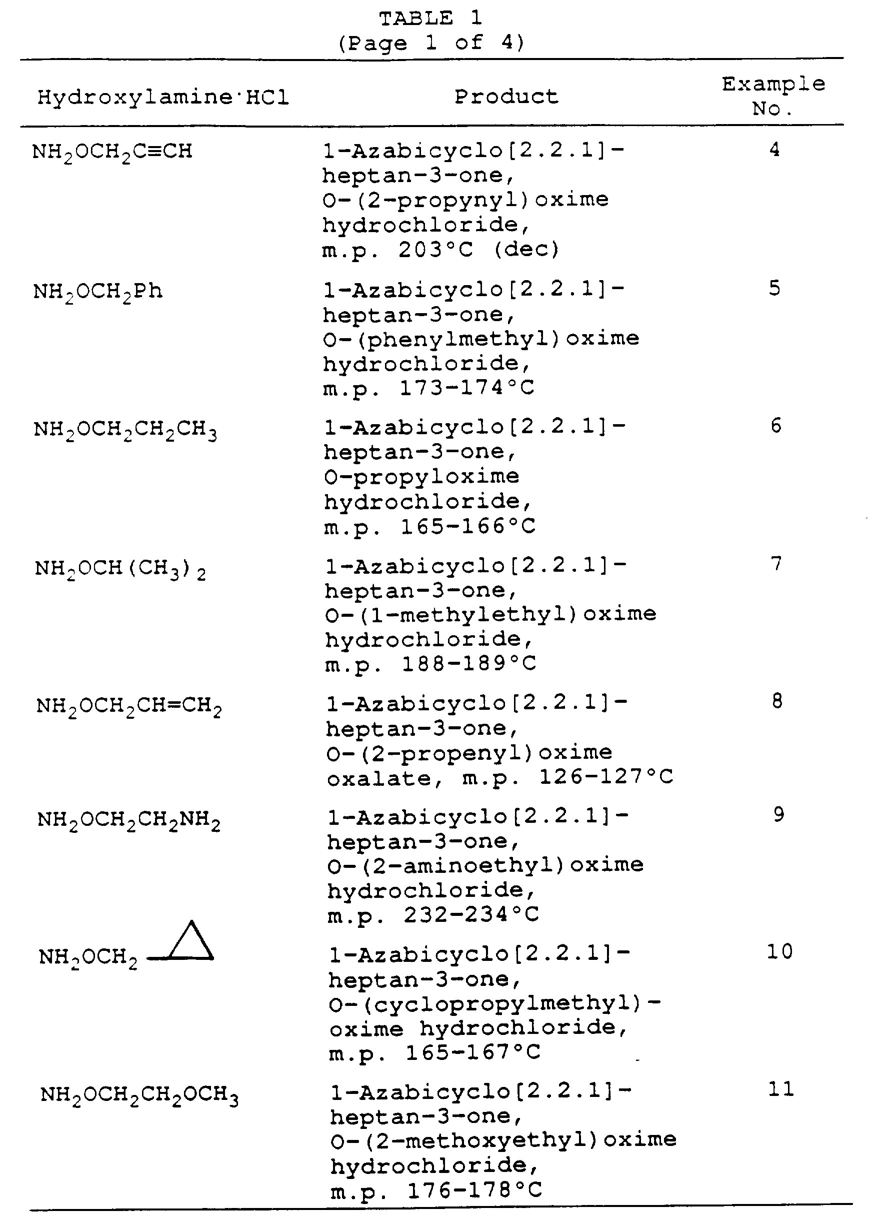

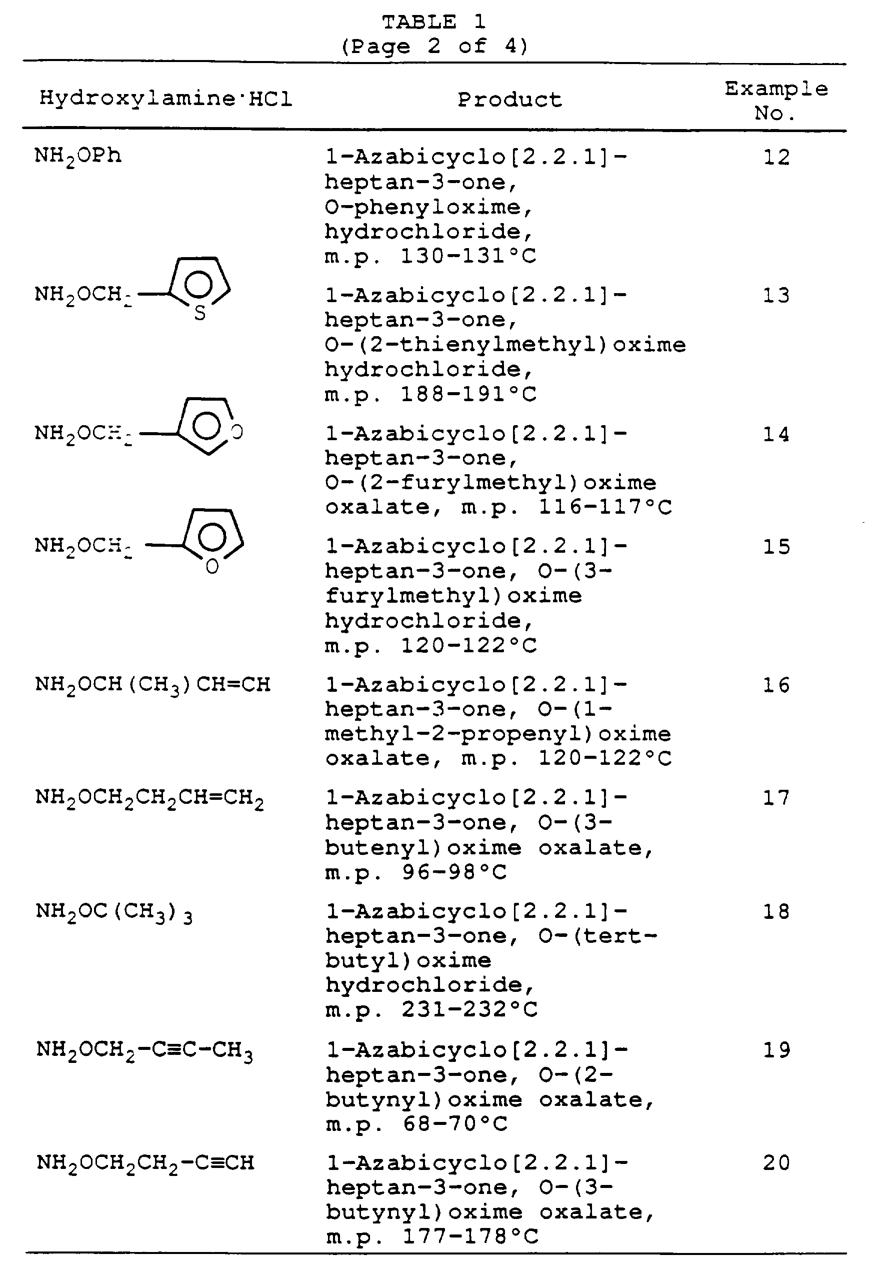

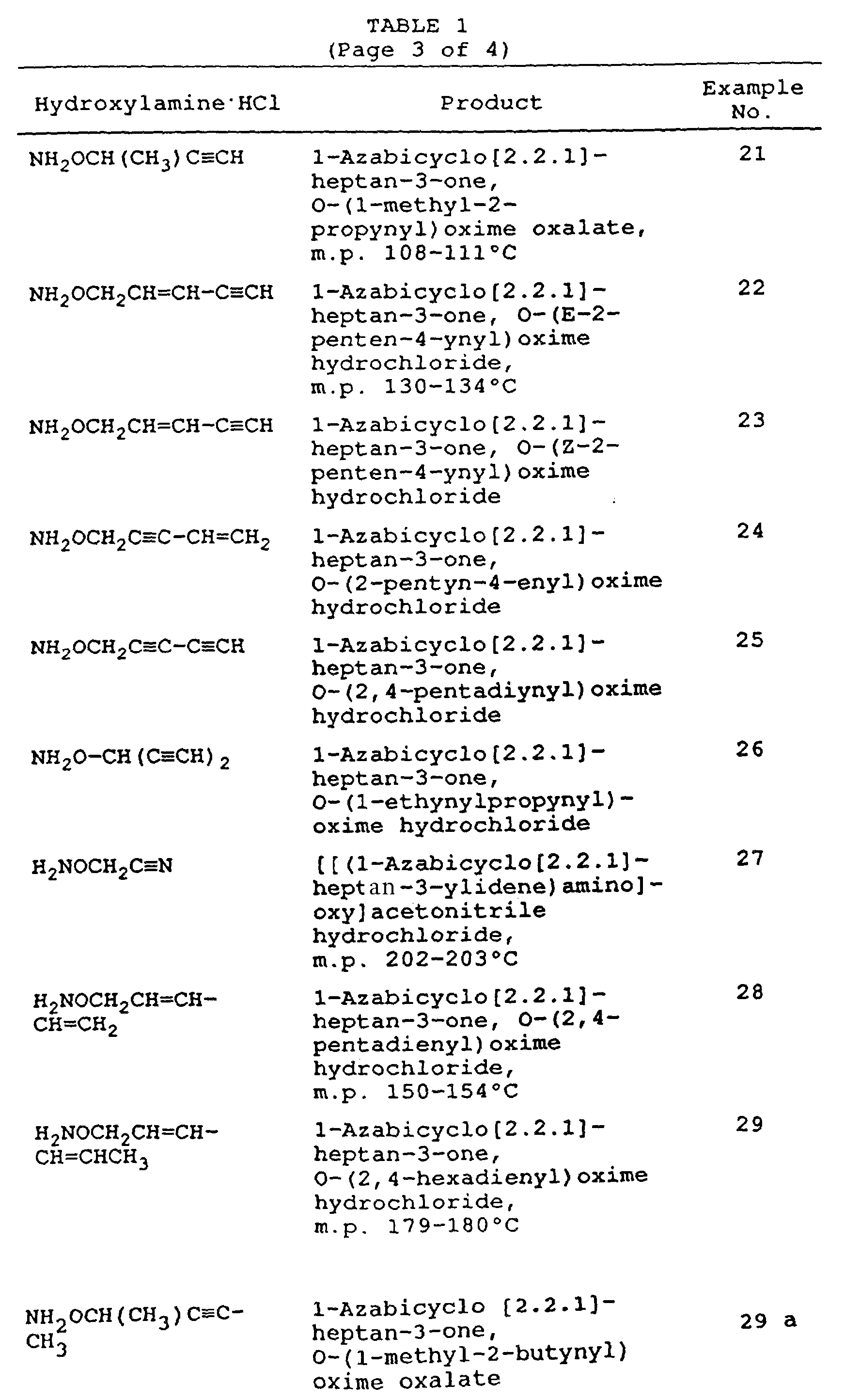

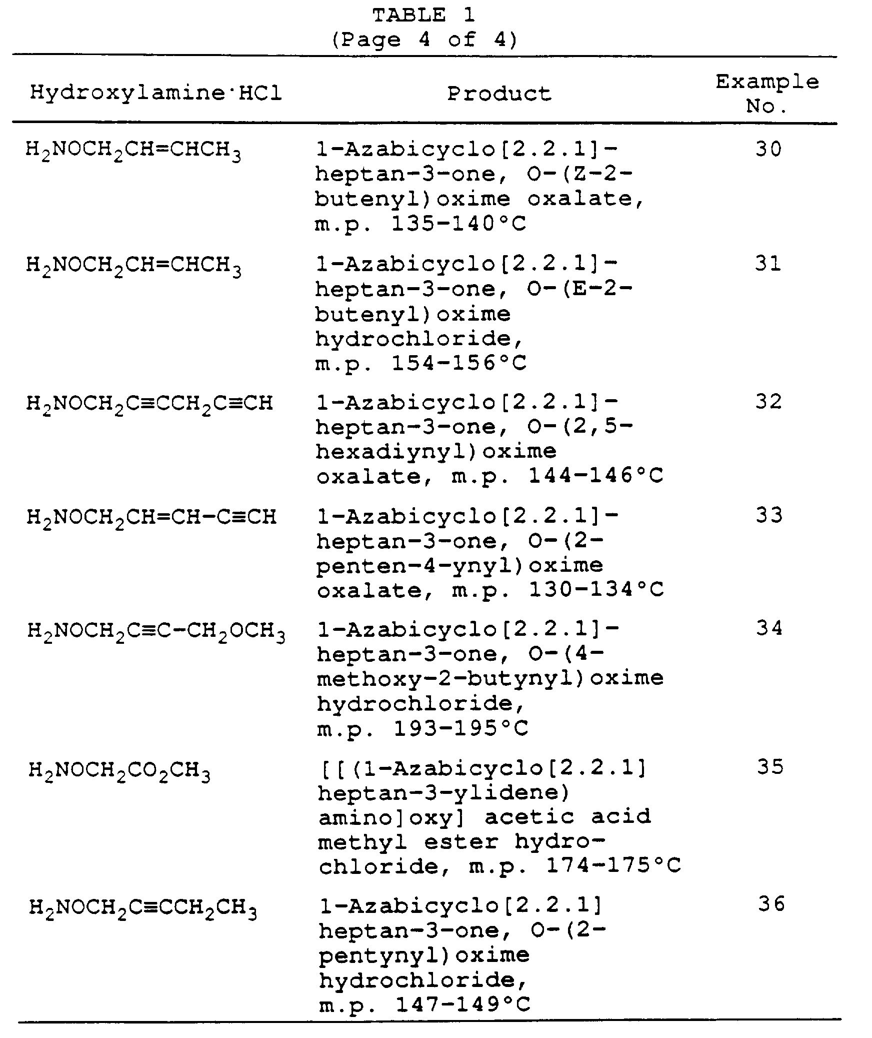

### EXAMPLE 37

### 1-Azabicyclo[2.2.2]octan-3-one, O-methyloxime

A mixture of the ketoamine˙HCl (5.56 g, 0.03 mole), 3-quinuclidinone (2.51 g, 0.03 mole), and potassium carbonate (11.0 g, 0.08 mole) in methanol (250 mL) was allowed to reflux for 2 hours. After removing the methanol under reduced pressure, the solution was suspended in water and extracted with ethyl ether (3 x 125 mL), dried over MgSO₄, filtered, and concentrated to give a mixture of syn and anti isomers. The oxalate salt was formed from the oil, m.p. 125-28°C.
NMR: δ (CDCl₃) 9.01 (br s, 2H), 4.03 (s, 1H), 3.89 (s, 1H), 3.80, 3.79 (two s, 3H), 3.27-3.12 (br m, 5H), 2.10-1.79 (br m, 4H). ₁₃C NMR: δ 164.7, 155.1, 154.6, 61.4, 59.4, 50.7, 50.1, 45.6, 45.5, 26.7, 22.5, 22.1, 21.4, 21.2 IR (cm⁻¹) 2361, 1748, 1693, 1652, 1463, 1403, 1320, 1221, 1103, 1048, 861, 832, 785, 721, 670.
Mass Spec.: 154, 137, 123, 108, 97, 82, 67, 55. C, H, N C₈ H₁₄N₂O˙1.25C₂H₂O₄

| | | | |
|---|---|---|---|
| Calc.: | C, 47.28; | H, 6.23; | N, 10.50. |
| Found: | C, 47.57; | H, 6.32; | N, 10.27. |

### EXAMPLE 38

### (E)- and (Z)- 1-Azabicyclo[2.2.2]octan-3-one, O-(2-propynyl)oxime

3-Quinuclidinone (1.5 g, 12 mmol) and 2-propynylhydroxylamine hydrochloride (1.29 g, 12 mmol) were dissolved in 20 mL of methanol and stirred at room temperature for 16 hours. The reaction was evaporated in vacuo to afford a dark brown semi-solid. The crude semi-solid was basified with aqueous K₂CO₃ and extracted with chloroform. The chloroform extract was evaporated to give the crude oxime as a mixture of the E- and Z-isomers. Separation of the isomers by chromatography (silica gel; dichloromethane-acetone-methanol (150:10:5), gave isomer A, 1.0 g (m.p. 81-83°C) and isomer B, 0.42 g (m.p. 100-101°C), respectively.
Isomer A:

| | | | | |
|---|---|---|---|---|
| C₁₀H₁₄N₂O: | Calc.: | C, 67.39; | H, 7.92; | N, 15.72. |
| | Found: | C, 67.26; | H, 7.94; | N, 15.59. |

Mass Spec.: m/e 178
¹H NMR: δ (CDCl₃) 1.7-1.85 (4H, m); 2.40-2.55 (1H, m), 2.58-2.65 (1H, m), 2.75-3.05 (4H, m), 3.63 (@H, s), 4.62 (2H, s). ₁₃C NMR: 6 (CDCl₃) 26.3, 28.6, 47.2, 52.3, 60.9, 74.2, 81.0, 166.0.
Isomer B:

| | | | | |
|---|---|---|---|---|
| C₁₀H₁₄N₂O: | Calc.: | C, 67.39; | H, 7.92; | N, 15.72. |
| | Found: | C, 66.95; | H, 8.02; | N, 15.59. |

Mass Spec.: m/e 178
¹H NMR: δ (CDCl₃) 1.6-1.8 (4H, m), 2.45-2.50 (1H, m), 2.78-3.0 (4H, m), 3.35-3.44 (1H, m), 3.45 (2H, s), 4.6 (2H, s). ₁₃C NMR: δ (CDCl₃) 23.5, 24.8, 47.2, 53.6, 60.8, 70.1, 81.0, 165.0.

### EXAMPLE 39

### 4-Piperidinone O-methyloxime hydrochloride

4-Piperidinone hydrochloride monohydrate (5 g, 32.5 mmol), methoxyamine hydrochloride (4.18 g, 50 mmol) and potassium carbonate (13.82 g, 100 mmol) were taken into 100 mL of ethanol and stirred at room temperature for 16 hours. The reaction mixture was filtered and the filtrate evaporated to afford a white solid. The crude solid was dissolved in water, made basic with solid potassium carbonate, and extracted with dichloromethane (3 x 100 mL). The combined extracts were dried over anhydrous sodium sulfate and evaporated in vacuo to afford a clear, colorless liquid. It was converted to its hydrochloride salt by treating with ethereal hydrogen chloride to give 233 g, m.p. 140-141°C.

| | | | | |
|---|---|---|---|---|
| C₆H₁₂N₂O˙HCl: | Calc.: | C, 43.77; | H, 7.96; | N, 17.02. |
| | Found: | C, 43.78; | H, 7.96; | N, 17.08 |

₁H NMR: δ (CDCl₃) 2.68-2.73 (t, 2H), 2.92-2.97 (t, 2H), 3.27-3.34 (m, 4H), 3.84 (s, 3H), 9.93 (br.s, 1H). ₁₃NMrR: δ (CDCl₃) 21.76, 28.05, 42.92, 44.29, 61.61, 150.07.
Mass Spec.: m/e 128.08 (M⁺ for free base).

### EXAMPLE 40

### 1-Methyl-4-piperidinone O-methyloxime hydrochloride

1-Methyl-4-piperidone (5 g, 44.2 mmol) and methoxyamine hydrochloride (3.69 g, 44.2 mmol) were taken into 25 mL of methanol and stirred at room temperature for 2 days. The reaction mixture was evaporated in vacuo to afford a solid residue. The residue was dissolved in water, made basic with solid potassium carbonate, and extracted with dichloromethane (3 x 100 mL). The combined extracts were dried over anhydrous sodium sulfate and evaporated to give a clear, yellow liquid that was purified on silica gel, eluting with dichloromethane-methanol (95:5) to afford 2.55 g of the desired product. Treatment with ethereal hydrogen chloride gave the hydrochloride salt, 2.99 g, m.p. 145-147°C.

| | | | | |
|---|---|---|---|---|
| C₇H₁₄N₂O˙HCl: | Calc.: | C, 47.06; | H, 8.46; | N, 15.68. |
| | Found: | C, 46.94; | H, 8.41; | N, 15.60. |

¹H NMR: δ (CDCl₃) 2.53-2.58 (m, 1H), 2.69-3.20 (m, 4H), 2.87-2.89 (d, 3H), 3.36-3.49 (m, 1H), 3.55-3.76 (m, 2H), 3.84 (s, 3H), 12.90 (br.s, 1H). ₁₃CCC NMR: δ (CDCl₃) 21.97, 28.25, 43.46, 53.09, 54.36, 61.64, 149.17.
Mass spec.: m/e 142.17 (M⁺) for free base).

### EXAMPLE 41

### Z-(±)-1-Azabicyclo[2.2.1]heptan-3-one O-2-propenyloxime oxalate and E-(±)-1-azabicyclo[2.2.1]-heptan-3-one O-2-propenyloxime oxalate

1-Azabicyclo[2.2.1]heptan-3-one (2 g, 18 mmol) and O-allylhydroxylamine hydrochloride hydrate (1.97 g, 18 mmol) were dissolved in 25 mL of methanol and stirred at room temperature for 18 hours. The reaction was evaporated in vacuo to give a viscous oil. The crude oil was dissolved in 50 mL of water, made basic with a saturated solution of potassium carbonate, and extracted with ether (3 x 100 mL). The combined extracts were dried over anhydrous sodium sulfate, and evaporated to give a clear, yellow liquid, which was purified on silica gel, eluting with dichloromethane-methanol (10:1). Isomer A, the less polar isomer, was isolated and converted to the oxalate salt to give 1.365 g, m.p. 130-132°C.

| | | | | |
|---|---|---|---|---|
| C₉H₁₄N₂O˙C₂H₂O₄: | Calc.: | C, 51.55; | H, 6.29; | N, 10.93. |
| | Found: | C, 51.52; | H, 6.37; | N, 10.88. |

¹H NMR: δ (DMSO) 12.0 (br.s, 2H), 5.86-6.02 (m, 1H), 5.17-5.31 (m, 2H), 4.5-4.52 (d, 2H), 3.85-4.06 (q, 2H), 3.16-3.42 (m, 5H), 2.14-2.27 (m, 1H), 1.70-1.75 (m, 1H).
Mass Spec.: m/e 166.1 (M⁺ for free base)
Isomer B, the more polar isomer, was converted to the oxalate to give 0.829 g, m.p. 102-114°C.

| | | | | |
|---|---|---|---|---|
| C₉H₁₄N₂O˙C₂H₂O₄: | Calc.: | C, 51.55; | H, 6.29; | N, 10.93. |
| | Found: | C, 51.02; | H, 6.22; | N, 10.63. |

¹H NMR: δ (DMSO) 11.2 (br.s, 2H), 5.88-6.04 (m, 1H), 5.17-5.33 (m, 2H), 4.50-4.55 (d, 2H), 3.73-4.00 (m, 3H), 3.11-3.42 (m, 4H), 2.08-2.21 (m, 1H), 1.62-1.66 (m, 1H).
Mass Spec.: m/e 166.1 (M⁺ for free base).

The above procedure was utilized for the synthesis of the following E and Z isomers:

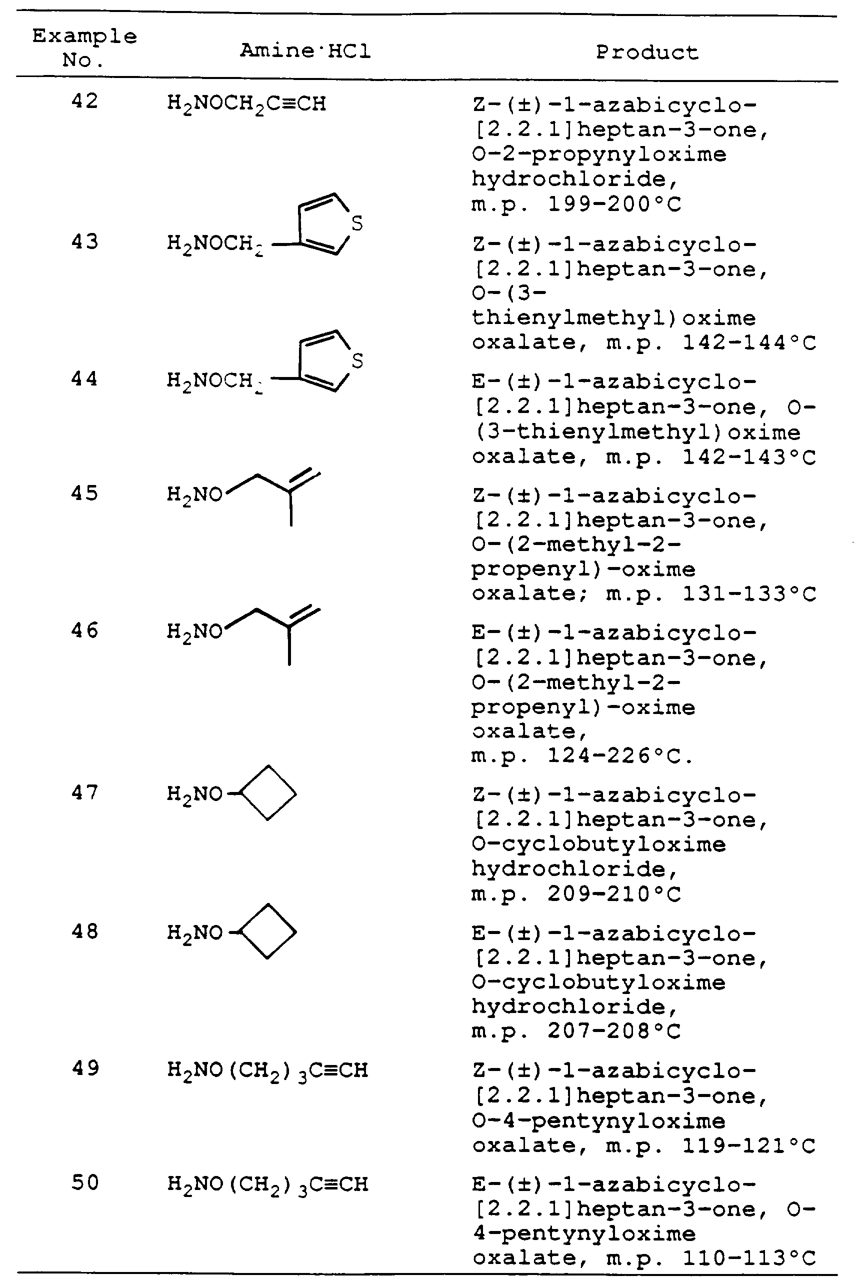

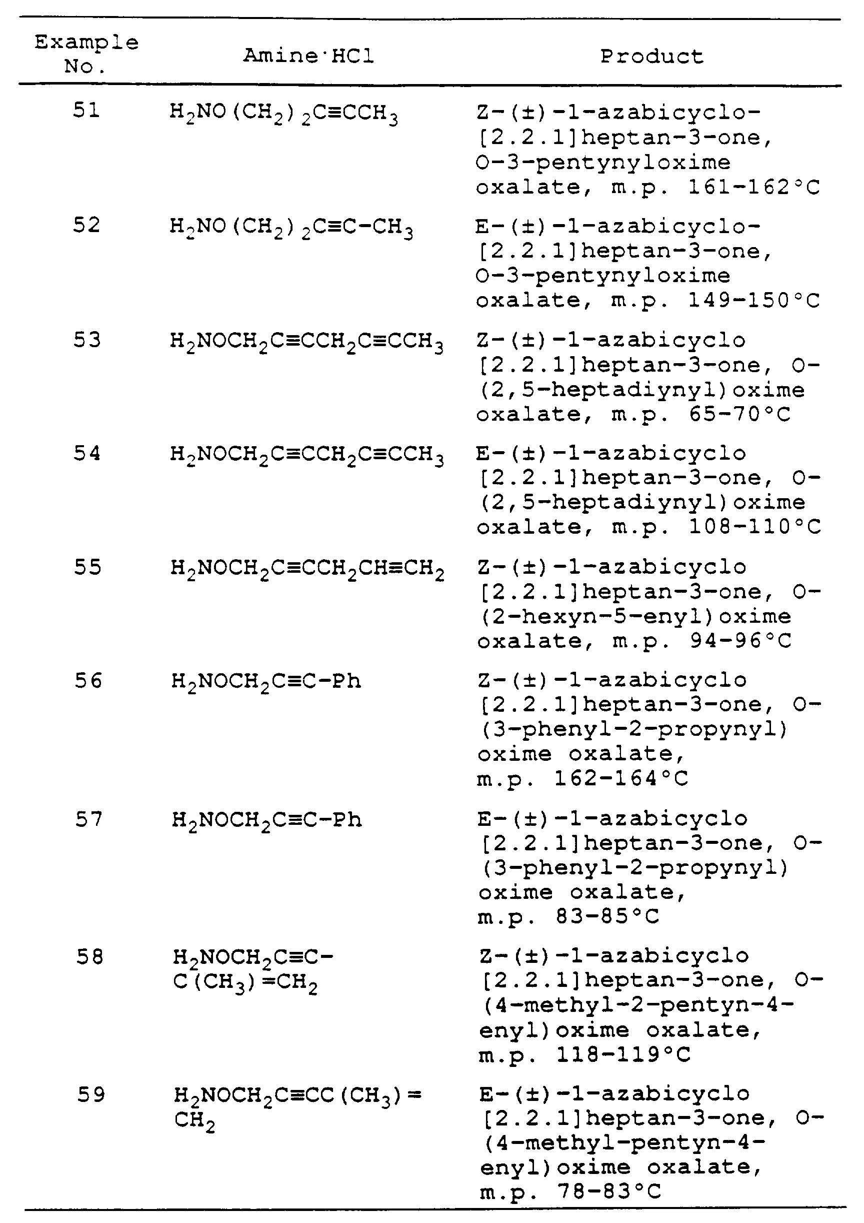

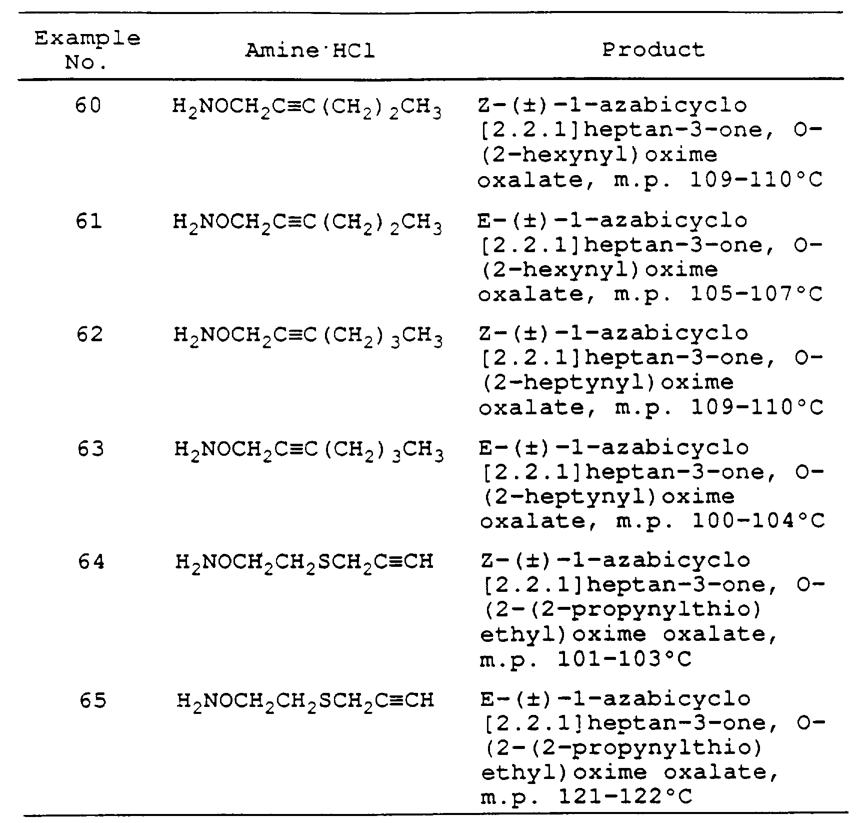

### EXAMPLE 66

### N-Methyl-8-Azabicyclo[3.2.1]octa-3-one methyl oxime hydrochloride

A solution of methoxyamine hydrochloride (4.18 g, 0.05 mol) in methanol was added to a rapidly stirring solution of N-methyl-8-azabicyclo[3.2.1]octa-3-one (commercially available) (6.96 g, 0.05 mol) in methanol while cooling with an ice-bath. The reaction mixture was allowed to warm up to room temperature, stirred at room temperature for 16 hours and then the solvent removed. The resulting solid residue was partitioned between CH₂Cl₂ and conc. aqueous K₂CO₃ solution. The organic layer was dried (K₂CO₃), and concentrated to give an oil. The oil was dissolved in ether and treated with ethereal-HCl. The resulting white solid ppt was separated by filtration and recrystallized from isopropanol-isopropyl ether to give 4.88 g (45%) of oxime hydrochloride as white solid: mp turns brown at 197°C to 200°C and melts with decomposition at 217°C and 220°C; ¹H NMR (D₂O) δ 4.710 (s, 4H), 4.013-3.939 (bd 1H). 3.765 (s, 3H), 3.259-3.194 (bd, 1H), 2.745 (s, 3H), 2.476-2.232 (m, 3H), 1.909-1.722 (m, 1H); ¹³C NMR (D₂O) δ 154.411, 66.306, 65.520, 64.257, 41.756, 38.930, 33.925, 27.461, 26.657; MS, m/e (relative intensity) 182 (0.15), 170 (8), 169 (71), 168 (64), 167 (9), 153 (2), 138 (13), 137 (97), 136 (4), 122 (7), 109 (7), 108 (11), 98 (3), 97 (16), 96 (71), 95 (12), 94 (26), 93 (3), 88 (4), 87 (72), 85 (2), 92 (100), 81 (24), 80 (7). Anal. Calcd. for C₉H₁₆N₂O·HCl: C, 52·80; H,8·37; N, 13·69. Found: C, 52·87; H, 8·31; N, 13·45.

### EXAMPLE 67

### N-Methyl-8-Azabicyclo[3.2.1]octan-2-one methyl oxime hydrochloride

The preparation of this compound is depicted in the Flow Chart set forth hereinafter and as described hereinbelow.
(a) Preparation of Ethyl 1,3,4,5-cycloheptatriene-1-carboxylate (2). Ethyl diazoacetate (43.4 g, 0.38 mol) was added slowly (syringe pump) over a period of 7 hours to a mixture of benzene (594.32 g, 7.61 mol) and tetrakis(perfluorobenzoato) dirhodium (II) (0.84 g, 00.16 mol). After stirring at room temperature for 16 hours, the solvent was removed and the oily residue distilled in vacuo to give 50.44 g (81%) of ethyl 1,3,5-cycloheptatriene-1-carboxylate: bp 90-96°C 2.4-3.0 mm Hg); ¹H NMR (CDCl₃) δ6.6 (m, 2H), 6.2 (m, 2H), 5.3 (m, 2H), 4.2 (q, 2H OCH₂CH₃), 2.5 (t, 1H), 1.3 (t, 3H CH₂CH₃).
(b) Preparation of N-Methyl Ethyl 8-Azabicyclo[3.2.1]oct-2-ene-2-carboxylate (3). A mixture of ethyl 1,3,5-cycloheptatriene-1-carboxylate (12.72 g, 0.077 mol), methylamine (23.13 g, 0.74 mol) and NaOH (3.1 g, 0.078 mol) in MeOH (60 mL) was autoclaved at 125°C for 26 hours. The reaction mixture was filtered, and the filtrate concentrated to give an oil. The oil was dissolved in MeOH (400 mL) and saturated with HCl gas; conc. sulphuric acid (8 mL) was added and the mixture refluxed for 12 hours. The oily residue obtained after evaporation of MeOH was partitioned between aqueous conc. Na₂CO₃ and ET₂O. The ether layer was evaporated as brown oil: IR (neat) 1720 (COOCH₃); ¹H (CDCl₃) δ6.8 (bt, 1H), 3.7 (s, 3H OCH₃), 2.35 (s, 3H NCH₃), 3.4-2.4 (m, 3H), 2.3-1.4 (m, 6H).
(c) Preparation of N-Methyl-2-acetyl-8-azabicyclo[3.2.1]oct-2-ene (4). To a mixture of ethyl trop-2-ene-2-carboxylate (3.43 g, 0.019 mol) and Me₃SiCl (10.32 g, 0.095 mol) in THF (70 mL) cooled to -100°C, MeLi (1.4 M, 17.07 mL, 0.0239 mol) was added dropwise. After warming up to room temperature, the reaction mixture was stirred for 16 hours. Excess Me₃SiCl and THF was evaporated off and the resulting residue was stirred with EtOH (6 mL) followed by water (6 mL). The mixture was then made acidic (6N HCl) and extracted with ether. The aqueous layer was made basic (solid K₂CO₃) and extracted with chloroform, dried (K₂CO₃) and concentrated to give 3.14 g (81%) of 2-acetyltrop-2-ene as an oil: IR (neat) 1670 (COCH₃); ¹H NMR (CDCl₃) δ6.7 (bt, 1H), 2.3 (s, NCH₃), 2.2 (s, C(≡O)CH₃).
(d) N-Methyl-2-Acetyl-8-azabicyclo[3.2.1]octane, compound (5) is obtained by catalytic reduction (H₂/RaNi) of compound (4) and is converted to N-Methyl-8-azabicyclo [3.2.1]octan-2-one, compound (7) by procedures known in the art, see J. Org. Chem. 55, 5025 (1990). In compound (6) TBDMSO stands for testing butyl dimethylsilyloxy. The ketone, compound (7) is converted to the methyloxime as generally described above in Example 66.

Similarly when N-methyl-2-acetyl-9-azabicyclo [4.2.1]nonane, which can be prepared from 2-acetyl-9-azabicyclo[4.2.1]non-2-ene which is commercially available, is substituted for N-methyl-2-acetyl-8-azabicyclo[3.2.1]octane, compound (5), N-methyl-8-azabicyclo[4.2.1]nonan-2-one methyl oxime is obtained.

The compounds of the present invention are centrally acting muscarinic agents and are thus useful as analgesic agents for the treatment of pain in mammals including an, as sleep aids, and as agents for treating the symptoms of senile dementia, Alzheimer's disease, Huntington's chorea, tardive dyskinesia, hyperkinesia, mania, or similar conditions of cerebral insufficiency characterized by decreased cerebral acetylcholine production or release.

The biological activity of compounds of the present invention was evaluated using a number of tests. The activity of compounds of this invention as central muscarinic binding site agonists and antagonists was measured. In the RQNB screening assay, which is described more fully by Mark Watson, et al, J. Pharmacol. and Exp. Ther., 237(2):411 (1986), rat cerebral cortex tissue was treated with radiolabeled quinuclidinyl benzilate, a known muscarinic binding site antagonist. The concentrations of test compound required to inhibit 50% of the binding of this muscarinic antagonist were then determined.

Similarly, in the RCMD screening assay, described more fully by T. W. Vickeroy, et al, 229(3):747 (1984), rat cerebral cortex tissue was treated with radiolabeled cis-methyldioxolane, a known muscarinic binding site agonist. The concentrations of test compounds required to inhibit 50% of the binding of this muscarinic agonist were then determined. These values are reported as IC₅₀ concentrations in Table 2 and demonstrate that the compounds of the present invention possess significant muscarinic activity.

**TABLE 2**

| Example No. | IC₅nM RCMD | IC₅₀nM RQNB |
|---|---|---|
| 3 | 18.2 | 24558 |
| 2 | 12.5 | |
| | | |
| | | 3112 |
| 4 | 3.5 | 2967 |
| 5 | 133 | 5947 |
| 6 | 120 | 3846 |
| 7 | 46 | 2030 |
| 8 | 53 | 4775 |
| 27 | 116 | 8820 |
| 28 (Isomer A) | 12.9 | 434 |
| 28 (Isomer B) | 256 | 9650 |

In therapeutic use as agents for treating pain or for treating cerebral insufficiency, the compounds utilized in the pharmaceutical method of this invention are administered to the patient at dosage levels of from 0.07 to 700 mg per day. For a normal human adult of approximately 70 kg of body weight, this translates into a dosage of from 0.01 to 100 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances which may also act is diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active compound is mixed with the carrier having the necessary binding properties in suitable proportions and compacted in the shape and size desired.

For preparing suppositories, a low-melting wax such as a mixture of fatty acid glycerides and cocoa butter is first melted, and the active ingredient is dispersed therein by, for example, stirring. The molten homogeneous mixture is then poured into convenient sized molds and allowed to cool and solidify.

Powders and tablets preferably contain between about 5% to about 70% by weight of the active ingredient. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is surrounded by a carrier, which is thus in association with it. In a similar manner, cachets are also included.

Tablets, powders, cachets, and capsules can be used as solid dosage forms suitable for oral administration.

Liquid form preparations include solutions suitable for oral or parenteral administration, or suspensions, and emulsions suitable for oral administration. Sterile water solutions of the active component or sterile solutions of the active component in solvents comprising water, ethanol, or propylene glycol may be mentioned as examples of liquid preparations suitable for parenteral administration.

Sterile solutions may be prepared by dissolving the active component in the desired solvent system, and then passing the resulting solution through a membrane filter to sterilize it or, alternatively, by dissolving the sterile compound in a previously sterilized solvent under sterile conditions.

Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethyl cellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can a packaged preparation, the package containing discrete quantities of the preparation, for example, packeted tablets, capsules, and powders in vials or ampoules. The unit dosage form can also be capsule, cachet, or tablet itself, or it can be the appropriate number of any of these packaged forms.

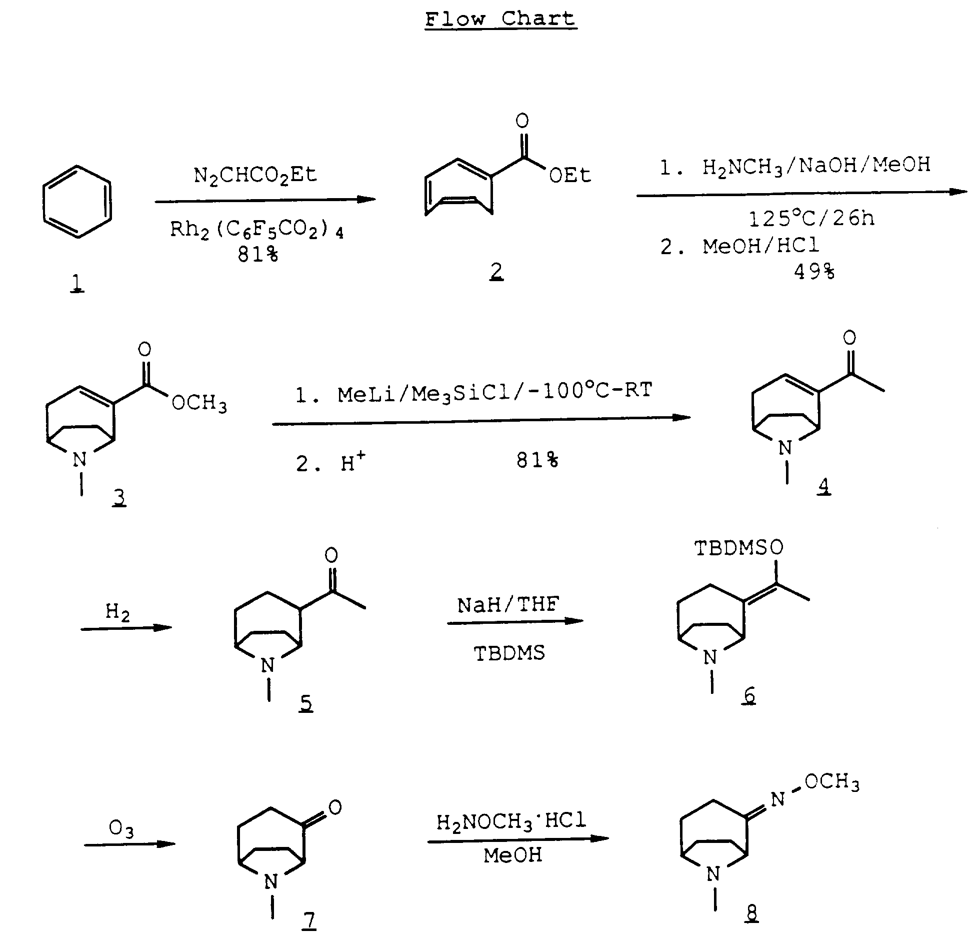

## Claims (Claims for the following Contracting State(s): AT, BE, CH, DE, DK, FR, GB, IT, LI, LU, NL, SE)

1. A compound of the following Formulas I, II, III, or IV, or a Z or E form thereof or a pharmaceutically acceptable salt thereof: 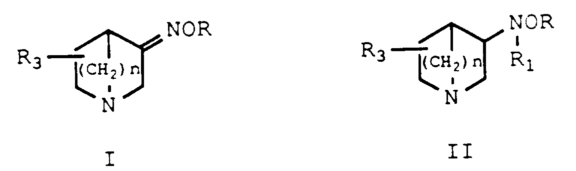 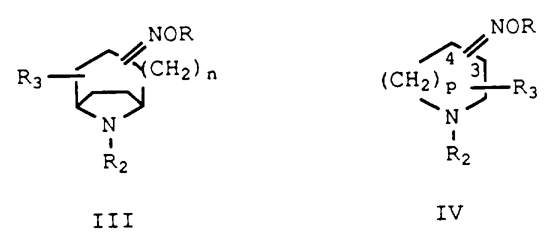
wherein n is one or two;
wherein p is zero or one to four;
wherein each of R₁ and R₂ is hydrogen or straight or branched lower alkyl having from 1 to 4 carbon atoms;
wherein R₃ is hydrogen, a straight or branched lower alkyl group having from 1 to 6 carbon atoms, hydroxy, a straight or branched lower alkoxy group having from 1 to 4 carbon atoms, acyloxy group wherein the acyl moiety has from 2 to 5 carbon atoms, or the group -(CH₂)_{q}-NR₈R₉ wherein q is zero or 1 to 4 and each of R₈ and R₉ is the same or different and is hydrogen or a straight or branched lower alkyl group having from 1 to 4 carbon atoms; or
cycloalkyl having from 3 to 6 carbon atoms;
wherein R is hydrogen: or the group:
-Y-Z
wherein Y is a bond or a straight or branched hydrocarbon chain having from 1 to 20 carbon atoms and optionally contains from 1 to 4 double and/or triple bonds; and Z is hydrogen;
a cycloalkyl group having from 3 to 6 carbon atoms;
an aromatic group selected from phenyl, 2- or 3-thienyl, 2- or 3-furanyl, 2- or 3-pyrrolyl wherein the aromatic group is unsubstituted or is substituted with 1 or 2 substituents selected from chlorine, bromine, fluorine, trifluoromethyl, nitro, amino, amino substituted with 1 or 2 straight or branched lower alkyl groups having from 1 to 4 carbon atoms, or straight or branched alkoxy having from 1 to 4 carbon atoms;
-NR₄R₅ wherein R₄ and R₅ are the same or different and are hydrogen or straight or branched alkyl having from 1 to 4 carbon atoms; 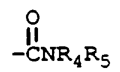
wherein R₄ and R₅ have the meanings defined above; 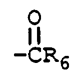
wherein R₆ is a straight or branched alkyl group having from 1 to 6 carbon atoms;
-CN;
-CO₂R₇ wherein R₇ is hydrogen, a straight or branched lower alkyl having from 1 to 6 carbon atoms and is straight or branched, or benzyl; or
-XR₁₀ where in X is oxygen or sulfur and R₁₀ is a straight or branched hydrocarbon chain having from 1 to 6 carbon atoms and which optionally contains 1 or 2 double or triple bonds;
with the proviso that the following compounds are excluded: (a) compounds of Formula I wherein n is two, and R is hydrogen, or the group Y-Z wherein Y is a bond and Z is -C(=O)NR₄R₅ or -C(=O)R₆; and
(b) compounds of Formulas III and IV wherein R is hydrogen
and pharmaceutically acceptable acid addition salts thereof.

2. A compound of Claim I having the formula 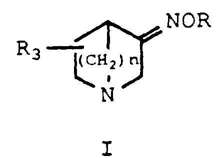 wherein n, R₃, and R have the meanings defined in Claim 1.

3. A compound of Claim 2 wherein n is one.

4. A compound of Claim 3 wherein R₃ is hydrogen and R is other than hydrogen.

5. A compound of Claim 4 which is
1-Azabicyclo[2.2.1]heptan-3-one, O-ethyloxime hydrochloride;
1-Azabicyclo[2.2.1]heptan-3-one, O-methyloxime hydrochloride;
1-Azabicyclo[2.2.1]heptan-3-one, O-2-propynyloxime hydrochloride;
1-Azabicyclo[2.2.1]heptan-3-one, O-(phenylmethyl)oxime hydrochloride;
1-Azabicyclo[2.2.1]heptan-3-one, O-propyloxime hydrochloride;
1-Azabicyclo[2.2.1]heptan-3-one, O-(1-methylethyl)oxime hydrochloride;
1-Azabicyclo[2.2.1]heptan-3-one, O-(2-propenyl)oxime oxalate;
1-Azabicyclo[2.2.1]heptan-3-one, O-(2-aminoethyl)oxime hydrochloride;
1-Azabicyclo[2.2.1]heptan-3-one, O-(cyclopropylmethyl)oxime hydrochloride;
1-Azabicyclo[2.2.1]heptan-3-one, O-(2-methoxyethyl)oxime hydrochloride;
1-Azabicyclo[2.2.1]heptan-3-one, O-phenyloxime hydrochloride;
1-Azabicyclo[2.2.1]heptan-3-one, O-(2-thienylmethyl)oxime hydrochloride;
1-Azabicyclo[2.2.1]heptan-3-one, O-(2-furylmethyl)oxime oxalate;
1-Azabicyclo[2.2.1]heptan-3-one, O-(3-furylmethyl)oxime hydrochloride;
1-Azabicyclo[2.2.1]heptan-3-one, O-(1-methyl-2-propenyl)oxime oxalate;
1-Azabicyclo[2.2.1]heptan-3-one, O-(3-butenyl)oxime oxalate;
1-Azabicyclo[2.2.1]heptan-3-one, O-(tert-butyl)oxime hydrochloride;
1-Azabicyclo[2.2.1]heptan-3-one, O-(2-butynyl)oxime oxalate;
1-Azabicyclo[2.2.1]heptan-3-one, O-(1-methyl-2-butynyl)oxime oxalate;
1-Azabicyclo[2.2.1]heptan-3-one, O-(1-methyl-2-propynyl)oxime oxalate;
1-Azabicyclo[2.2.1]heptan-3-one, O-(E-2-penten-4-ynyl)oxime hydrochloride;
1-Azabicyclo[2.2.1]heptan-3-one, O-(Z-2-penten-4-ynyl)oxime hydrochloride;
1-Azabicyclo[2.2.1]heptan-3-one, O-(2-pentyn-4-enyl)oxime hydrochloride;
1-Azabicyclo[2.2.1]heptan-3-one, 0-(2,4-pentadiynyl)oxime hydrochloride;
1-Azabicyclo[2.2.1]heptan-3-one, O-(1-ethynylpropynyl)oxime hydrochloride;
[1-Azabicyclo[2.2.1]heptane-3-ylidene)amino]oxyacetonitrile hydrochloride;
1-Azabicyclo[2.2.1]heptan-3-one, O-(2,4-pentadienyl)oxime hydrochloride;
1-Azabicyclo[2.2.1]heptan-3-one, O-(2,4-hexadienyl)oxime hydrochloride;
1-AzabicycIo[2.2.1]heptan-3-one, O-(Z-2-butenyl)oxime oxalate;
1-Azabicyclo[2.2.1]heptan-3-one, O-(E-2-butenyl)oxime hydrochloride;
1-Azabicyclo[2.2.1]heptan-3-one, O-(2,5-hexadiynyl)oxime oxalate;
1-Azabicyclo[2.2.1]heptan-3-one, O-(2-penten-4-ynyl)oxime oxalate;
1-Azabicyclo[2.2.1]heptan-3-one, 0-(4-methoxy-2-butynyl)oxime hydrochloride;
[[(1-Azabicyclo[2.2.1]heptan-3-ylidene) amino]oxy]acetic acid methylester hydrochloride;
1-Azabicyclo[2.2.1]heptan-3-one, O-(2-pentynyl)oxime hydrochloride;
Z-(±)-1-Azabicyclo[2.2.1]heptan-3-one, O-2-propenyloxime oxalate;
E-(±)-1-Azabicyclo[2.2.1]heptan-3-one, O-2-propenyloxime oxalate;
Z-(±)-1-Azabicyclo[2.2.1]heptan-3-one, O-2-propynyloxime hydrochloride;
Z-(±)-1-Azabicyclo[2.2.1]heptan-3-one, O-(3-thienylmethyl)oxime oxalate;
E-(±)-1-Azabicyclo[2.2.1]heptan-3-one, O-(3-thienylmethyl)oxime oxalate;
Z-(±)-1-Azabicyclo[2.2.1]heptan-3-one, O-(2-methyl-2-propenyl)oxime oxalate;
E-(±)-1-Azabicyclo[2.2.1]heptan-3-one, O-(2-methyl-2-propenyl)oxime oxalate;
Z-(±)-1-Azabicyclo[2.2.1]heptan-3-one, O-cyclobutyloxime hydrochloride;
E-(±)-1-Azabicyclo[2.2.1]heptan-3-one, O-cyclobutyloxime hydrochloride;
Z-(±)-1-Azabicyclo[2.2.1]heptan-3-one, O-4-pentynyloxime oxalate;
E-(±)-1-Azabicyclo[2.2.1]heptan-3-one, O-4-pentynyloxime oxalate;
Z-(±)-1-Azabicyclo[2.2.1]heptan-3-one, O-3-pentynyloxime oxalate;
E-(±)-1-Azabicyclo[2.2.1]heptan-3-one, O-3-pentynyloxime oxalate;
Z-(±)-1-azabicyclo[2.2.1]heptan-3-one, O-(2,5-heptadiynyl)oxime oxalate;
E-(±)-1-azabicyclo[2.2.1]heptan-3-one, O-(2,5-heptadiynyl)oxime oxalate;
Z-(±)-1-azabicyclo[2.2.1]heptan-3-one, O-(2-hexyn-5-enyl)oxime oxalate;
Z-(±)-1-azabicyclo[2.2.1]heptan-3-one, O-(3-phenyl-2-propynyl)oxime oxalate;
E-(±)-1-azabicyclo[2.2.1]heptan-3-one, O-(3-phenyl-2-propynyl)oxime oxalate;
Z-(±)-1-azabicyclo[2.2.1]heptan-3-one, O-(4-methyl-2-pentyn-4-enyl)oxime oxalate;
E-(±)-1-azabicyclo[2.2.1]heptan-3-one, O-(4-methylpentyn-4-enyl)oxime oxalate;
Z-(±)-1-azabicyclo[2.2.1]heptan-3-one, O-(2-hexynyl)oxime oxalate;
E-(±)-1-azabicyclo[2.2.1]heptan-3-one, O-(2-hexynyl)oxime oxalate;
Z-(±)-1-azabicyclo[2.2.1]heptan-3-one, O-(2-heptynyl)oxime oxalate;
E-(±)-1-azabicyclo[2.2.1]heptan-3-one, O-(2-heptynyl)oxime oxalate;
Z-(±)-1-azabicyclo[2.2.1]heptan-3-one, O-(2-(2-propynylthio)ethyl)oxime oxalate; and
E-(±)-1-azabicyclo[2.2.1]heptan-3-one, O-(2-2-propynylthio)ethyl)oxime oxalate.

6. A compound of Claim 2 wherein n is 2.

7. A compound of Claim 6 wherein R₃ is hydrogen and R is other than hydrogen.

8. A compound of Claim 7 which is
1-Azabicyclo[2.2.2]octan-3-one, O-ethyloxime hydrochloride;
1-Azabicyclo[2.2.2]octan-3-one, O-methyloxime,
1-Azabicyclo[2.2.2]octan-3-one, O-2-(propynyl)oxime;
1-Azabicyclo[2.2.2]octan-3-one, O-(phenylmethyl)oxime hydrochloride;
1-Azabicyclo[2.2.2]octan-3-one, O-propyloxime hydrochloride;
1-Azabicyclo[2.2.2]octan-3-one, O-(1-methylethyl)oxime hydrochloride;
1-Azabicyclo[2.2.2]octan-3-one, O-(2-propenyl)oxime oxalate;
1-Azabicyclo[2.2.2]octan-3-one, O-(2-aminoethyl)oxime hydrochloride;
1-Azabicyclo[2.2.2]octan-3-one, O-(cyclopropylmethyl)oxime hydrochloride;
1-Azabicyclo[2.2.2]octan-3-one, O-(2-methoxyethyl)oxime hydrochloride;
1-Azabicyclo[2.2.2]octan-3-one, O-phenyloxime hydrochloride;
1-Azabicyclo[2.2.2]octan-3-one, O-(2-thienylmethyl)oxime hydrochloride;
1-Azabicyclo[2.2.2]octan-3-one, O-(2-furylmethyl)oxime oxalate;
1-Azabicyclo[2.2.2]octan-3-one, O-(3-furylmethyl)oxime hydrochloride;
1-Azabicyclo[2.2.2]octan-3-one, O-(1-methyl-2-propenyl)oxime oxalate;
1-Azabicyclo[2.2.2]octan-3-one, O-(3-butenyl)oxime oxalate;
1-Azabicyclo[2.2.2]octan-3-one, O-(tert-butyl)oxime hydrochloride;
1-Azabicyclo[2.2.2]octan-3-one, O-(2-butynyl)oxime oxalate;
1-Azabicyclo[2.2.2]octan-3-one, O-(1-methyl-2-propynyl)oxime oxalate;
1-Azabicyclo[2.2.2]octan-3-one, O-(1-methyl-2-butynyl)oxime oxalate;
1-Azabicyclo[2.2.2]octan-3-one, O-(E-2-penten-4-ynyl)oxime hydrochloride;
1-Azabicyclo[2.2.2]octan-3-one, O-(Z-2-penten-4-ynyl)oxime hydrochloride;
1-Azabicyclo[2.2.2]octan-3-one, O-(2-pentyn-4-enyl)oxime hydrochloride;
1-Azabicyclo[2.2.2]octan-3-one, O-(2,4-pentadiynyl)oxime hydrochloride; and
1-Azabicyclo[2.2.2]octan-3-one, O-(1-ethynylpropynyl)oxime hydrochloride.

9. A compound of Claim 1 which is 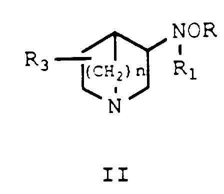 wherein n, R₃, R₁, and R have the meanings defined in Claim 1.

10. A compound of Claim 1 having the formula: 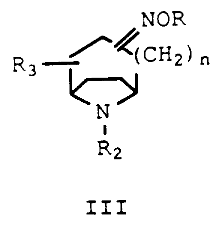 wherein n, R, R₂, and R₃ have the meanings defined in Claim 1.

11. A compound of Claim 10 wherein n is one.

12. A compound of Claim 11 which is N-methyl-8-azabicyclo[3.2.1]octa-3-one methyl oxime hydrochloride or N-methyl-8-azabicyclo[3.2.1] octa-2-one methyloxime hydrochloride.

13. A compound of Claim 1 having the formula: 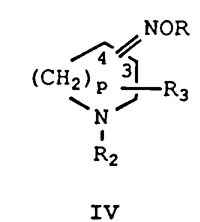 wherein p, R, R₂, and R₃ have the meanings defined in Claim 1.

14. A compound of Claim 13 wherein p is 2.

15. A compound of Claim 14 which is 4-piperidinone O-methyloxime hydrochloride; or 1-methyl-4-piperidinone O-methyloxime hydrochloride.

16. A pharmaceutical composition comprising a compound of Claims 1 to 15 together with a pharmaceutically acceptable carrier.

17. Use of a compound according to Claims 1 to 15 for the preparation of a pharmaceutical composition for alleviating pain or treating the symptoms of senile cognitive decline in a patient.

18. A method for preparing a compound of Claims 1 to 15 which comprises to obtain a compound of Formula I, III, or IV, respectively, reacting a ketone selected from the formula 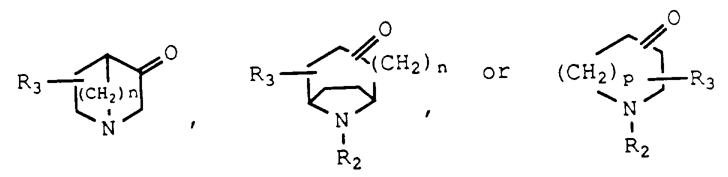 with an amine hydrochloride of the formula NH₂OR·HCl wherein n, p, R₃, R₂, and R have the meanings defined in Claim 1 in methanol at room temperature and to obtain compounds of Formula II, reducing the compounds of Formula I, and, if desired, forming a pharmaceutically acceptable salt with an organic or anorganic acid.

## Claims (Claims for the following Contracting State(s): ES, GR)

1. A process for the preparation of a compound of the following Formulas I, II, III, or IV, or a Z or E form thereof or a pharmaceutically acceptable salt thereof: 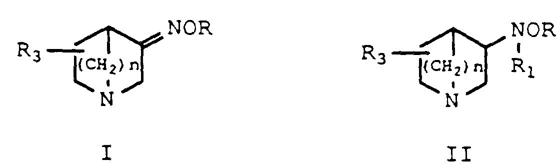 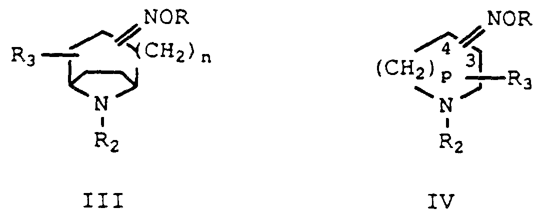
wherein n is one or two;
wherein p is zero or one to four;
wherein each of R₁ and R₂ is hydrogen or straight or branched lower alkyl having from 1 to 4 carbon atoms;
wherein R₃ is hydrogen, a straight or branched lower alkyl group having from 1 to 6 carbon atoms, hydroxy, a straight or branched lower alkoxy group having from 1 to 4 carbon atoms, acyloxy group wherein the acyl moiety has from 2 to 5 carbon atoms, or the group -(CH₂)_{q}-NR₈R₉ wherein q is zero or 1 to 4 and each of R₈ and R₉ is the same or different and is hydrogen or a straight or branched lower alkyl group having from 1 to 4 carbon atoms; or
cycloalkyl having from 3 to 6 carbon atoms;
wherein R is hydrogen: or the group:
-Y-Z
wherein Y is a bond or a straight or branched hydrocarbon chain having from 1 to 20 carbon atoms and optionally contains from 1 to 4 double and/or triple bonds; and Z is hydrogen;
a cycloalkyl group having from 3 to 6 carbon atoms;
an aromatic group selected from phenyl, 2- or 3-thienyl, 2- or 3-furanyl, 2- or 3-pyrrolyl wherein the aromatic group is unsubstituted or is substituted with 1 or 2 substituents selected from chlorine, bromine, fluorine, trifluoromethyl, nitro, amino, amino substituted with 1 or 2 straight or branched lower alkyl groups having from 1 to 4 carbon atoms, or straight or branched alkoxy having from 1 to 4 carbon atoms;
-NR₄R₅ wherein R₄ and R₅ are the same or different and are hydrogen or straight or branched alkyl having from 1 to 4 carbon atoms; 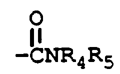
wherein R₄ and R₅ have the meanings defined above; 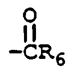
wherein R₆ is a straight or branched alkyl group having from 1 to 6 carbon atoms;
-CN;
-CO₂R₇ wherein R₇ is hydrogen, a straight or branched lower alkyl having from 1 to 6 carbon atoms and is straight or branched, or benzyl; or
-XR₁₀ where in X is oxygen or sulfur and R₁₀ is a straight or branched hydrocarbon chain having from 1 to 6 carbon atoms and which optionally contains 1 or 2 double or triple bonds;
with the proviso that the following compounds are excluded: (a) compounds of Formula I wherein n is two, and R is hydrogen, or the group Y-Z wherein Y is a bond and Z is -C(=O)NR₄R₅ or -C(=O)R₆; and
(b) compounds of Formulas III and IV wherein R is hydrogen,
and pharmaceutically acceptable acid addition salts thereof which comprises to obtain a compound of Formula I, III, or IV, respectively, reacting a ketone selected from the formula 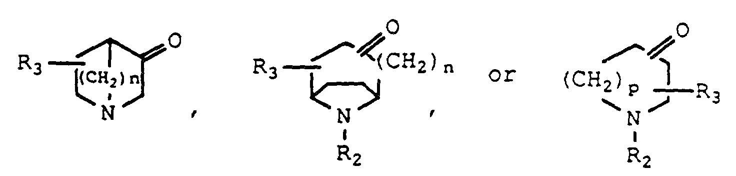 with an amine hydrochloride of the formula NH₂OR·HCl wherein n, p, R₃, R₂, and R have the meanings defined above in methanol at room temperature and to obtain compounds of Formula II, reducing the compounds of Formula I and, if desired, forming a pharmaceutically acceptable salt with an organic or anorganic acid.

2. A process according to Claim 1 for the preparation of compounds of Claim 1 having the formula 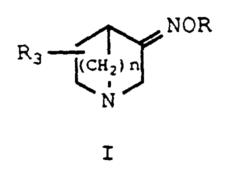 wherein n, R₃, and R have the meanings defined in Claim 1.

3. A process according to Claim 1 for the preparation of compounds of Claim 2 wherein n is one

4. A process according to Claim 1 for the preparation of compounds of Claim 3 wherein R₃ is hydrogen and R is other than hydrogen.

5. A process according to Claim 1 for the preparation of compounds of Claim 4 which is
1-Azabicyclo[2.2.1]heptan-3-one, O-ethyloxime hydrochloride;
1-Azabicyclo[2.2.1]heptan-3-one, O-methyloxime hydrochloride;
1-Azabicyclo[2.2.1]heptan-3-one, O-2-propynyloxime hydrochloride;
1-Azabicyclo[2.2.1]heptan-3-one, O-(phenylmethyl)oxime hydrochloride;
1-Azabicyclo[2.2.1]heptan-3-one, O-propyloxime hydrochloride;
1-Azabicyclo[2.2.1]heptan-3-one, O-(1-methylethyl)oxime hydrochloride;
1-Azabicyclo[2.2.1]heptan-3-one, O-(2-propenyl)oxime oxalate;
1-Azabicyclo[2.2.1]heptan-3-one, O-(2-aminoethyl)oxime hydrochloride;
1-Azabicyclo[2.2.1]heptan-3-one, O-(cyclopropylmethyl)oxime hydrochloride;
1-Azabicyclo[2.2.1]heptan-3-one, O-(2-methoxyethyl)oxime hydrochloride;
1-Azabicyclo[2.2.1]heptan-3-one, O-phenyloxime hydrochloride;
1-Azabicyclo[2.2.1]heptan-3-one, O-(2-thienylmethyl)oxime hydrochloride;
1-Azabicyclo[2.2.1]heptan-3-one, O-(2-furylmethyl)oxime oxalate;
1-Azabicyclo[2.2.1]heptan-3-one, O-(3-furylmethyl)oxime hydrochloride;
1-Azabicyclo[2.2.1]heptan-3-one, O-(1-methyl-2-propenyl)oxime oxalate;
1-Azabicyclo[2.2.1]heptan-3-one, O-(3-butenyl)oxime oxalate;
1-Azabicyclo[2.2.1]heptan-3-one, O-(tert-butyl)oxime hydrochloride;
1-Azabicyclo[2.2.1]heptan-3-one, O-(2-butynyl)oxime oxalate;
1-Azabicyclo[2.2.1]heptan-3-one, O-(1-methyl-2-butynyl)oxime oxalate;
1-Azabicyclo[2.2.1]heptan-3-one, O-(1-methyl-2-propynyl)oxime oxalate;
1-Azabicyclo[2.2.1]heptan-3-one, O-(E-2-penten-4-ynyl)oxime hydrochloride;
1-Azabicyclo[2.2.1]heptan-3-one, O-(Z-2-penten-4-ynyl)oxime hydrochloride;
1-Azabicyclo[2.2.1]heptan-3-one, O-(2-pentyn-4-enyl)oxime hydrochloride;
1-Azabicyclo[2.2.1]heptan-3-one, O-(2,4-pentadiynyl)oxime hydrochloride;
1-Azabicyclo[2.2.1]heptan-3-one, O-(1-ethynylpropynyl)oxime hydrochloride;
[1-Azabicyclo[2.2.1]heptane-3-ylidene)amino]oxyacetonitrile hydrochloride;
1-Azabicyclo[2.2.1]heptan-3-one, O-(2,4-pentadienyl)oxime hydrochloride;
1-Azabicyclo[2.2.1]heptan-3-one, O-(2,4-hexadienyl)oxime hydrochloride;
1-Azabicyclo[2.2.1]heptan-3-one, O-(Z-2-butenyl)oxime oxalate;
1-Azabicyclo[2.2.1]heptan-3-one, O-(E-2-butenyl)oxime hydrochloride;
1-Azabicyclo[2.2.1]heptan-3-one, O-(2,5-hexadiynyl)oxime oxalate;
1-Azabicyclo[2.2.1]heptan-3-one, O-(2-penten-4-ynyl)oxime oxalate;
1-Azabicyclo[2.2.1]heptan-3-one, O-(4-methoxy-2-butynyl)oxime hydrochloride;
[[(1-Azabicyclo[2.2.1]heptan-3-ylidene) amino]oxy]acetic acid methylester hydrochloride;
1-Azabicyclo[2.2.1]heptan-3-one, O-(2-pentynyl)oxime hydrochloride;
Z-(±)-1-Azabicyclo[2.2.1]heptan-3-one, O-2-propenyloxime oxalate;
E-(±)-1-Azabicyclo[2.2.1]heptan-3-one, O-2-propenyloxime oxalate;
Z-(±)-1-Azabicyclo[2.2.1]heptan-3-one, O-2-propynyloxime hydrochloride;
Z-(±)-1-Azabicyclo[2.2.1]heptan-3-one, O-(3-thienylmethyl)oxime oxalate;
E-(±)-1-Azabicyclo[2.2.1]heptan-3-one, O-(3-thienylmethyl)oxime oxalate;
Z-(±)-1-Azabicyclo[2.2.1]heptan-3-one, O-(2-methyl-2-propenyl)oxime oxalate;
E-(±)-1-Azabicyclo[2.2.1]heptan-3-one, O-(2-methyl-2-propenyl)oxime oxalate;
Z-(±)-1-Azabicyclo[2.2.1]heptan-3-one, O-cyclobutyloxime hydrochloride;
E-(±)-1-Azabicyclo[2.2.1]heptan-3-one, O-cyclobutyloxime hydrochloride;
Z-(±)-1-Azabicyclo[2.2.1]heptan-3-one, O-4-pentynyloxime oxalate;
E-(±)-1-Azabicyclo[2.2.1]heptan-3-one, O-4-pentynyloxime oxalate;
Z-(±)-1-Azabicyclo[2.2.1]heptan-3-one, O-3-pentynyloxime oxalate;
E-(±)-1-Azabicyclo[2.2.1]heptan-3-one, O-3-pentynyloxime oxalate;
Z-(±)-1-azabicyclo[2.2.1]heptan-3-one, O-(2,5-heptadiynyl)oxime oxalate;
E-(±)-1-azabicyclo[2.2.1]heptan-3-one, O-(2,5-heptadiynyl)oxime oxalate;
Z-(±)-1-azabicyclo[2.2.1]heptan-3-one, O-(2-hexyn-5-enyl)oxime oxalate;
Z-(±)-1-azabicyclo[2.2.1]heptan-3-one, O-(3-phenyl-2-propynyl)oxime oxalate;
E-(±)-1-azabicyclo[2.2.1]heptan-3-one, O-(3-phenyl-2-propynyl)oxime oxalate;
Z-(±)-1-azabicyclo[2.2.1]heptan-3-one, O-(4-methyl-2-pentyn-4-enyl)oxime oxalate;
E-(±)-1-azabicyclo[2.2.1]heptan-3-one, O-(4-methylpentyn-4-enyl)oxime oxalate;
Z-(±)-1-azabicyclo[2.2.1]heptan-3-one, O-(2-hexynyl)oxime oxalate;
E-(±)-1-azabicyclo[2.2.1]heptan-3-one, O-(2-hexynyl)oxime oxalate;
Z-(±)-1-azabicyclo[2.2.1]heptan-3-one, O-(2-heptynyl)oxime oxalate;
E-(±)-1-azabicyclo[2.2.1]heptan-3-one, O-(2-heptynyl)oxime oxalate;
Z-(±)-1-azabicyclo[2.2.1]heptan-3-one, O-(2-(2-propynylthio)ethyl)oxime oxalate; and
E-(±)-1-azabicyclo[2.2.1]heptan-3-one, O-(2-2-propynylthio)ethyl)oxime oxalate.

6. A process according to Claim 1 for the preparation of compounds of Claim 2 wherein n is 2.

7. A process according to Claim 1 for the preparation of compounds of Claim 6 wherein R₃ is hydrogen and R is other than hydrogen

8. A process according to Claim 1 for the preparation of compounds of Claim 7 which is
1-Azabicyclo[2.2.2]octan-3-one, O-ethyloxime hydrochloride;
1-Azabicyclo[2.2.2]octan-3-one, O-methyloxime,
1-Azabicyclo[2.2.2]octan-3-one, O-2-(propynyl)oxime;
1-Azabicyclo[2.2.2]octan-3-one, O-(phenylmethyl)oxime hydrochloride;
1-Azabicyclo[2.2.2]octan-3-one, O-propyloxime hydrochloride;
1-Azabicyclo[2.2.2]octan-3-one, O-(1-methylethyl)oxime hydrochloride;
1-Azabicyclo[2.2.2]octan-3-one, O-(2-propenyl)oxime oxalate;
1-Azabicyclo[2.2.2]octan-3-one, O-(2-aminoethyl)oxime hydrochloride;
1-Azabicyclo[2.2.2]octan-3-one, O-(cyclopropylmethyl)oxime hydrochloride;
1-Azabicyclo[2.2.2]octan-3-one, O-(2-methoxyethyl)oxime hydrochloride;
1-Azabicyclo[2.2.2]octan-3-one, O-phenyloxime hydrochloride;
1-Azabicyclo[2.2.2]octan-3-one, O-(2-thienylmethyl)oxime hydrochloride;
1-Azabicyclo[2.2.2]octan-3-one, O-(2-furylmethyl)oxime oxalate;
1-Azabicyclo[2.2.2]octan-3-one, O-(3-furylmethyl)oxime hydrochloride;
1-Azabicyclo[2.2.2]octan-3-one, O-(1-methyl-2-propenyl)oxime oxalate;
1-Azabicyclo[2.2.2]octan-3-one, O-(3-butenyl)oxime oxalate;
1-Azabicyclo[2.2.2]octan-3-one, O-(tert-butyl)oxime hydrochloride;
1-Azabicyclo[2.2.2]octan-3-one, O-(2-butynyl)oxime oxalate;
1-Azabicyclo[2.2.2]octan-3-one, O-(1-methyl-2-propynyl)oxime oxalate;
1-Azabicyclo[2.2.2]octan-3-one, O-(1-methyl-2-butynyl)oxime oxalate;
1-Azabicyclo[2.2.2]octan-3-one, O-(E-2-penten-4-ynyl)oxime hydrochloride;
1-Azabicyclo[2.2.2]octan-3-one, O-(Z-2-penten-4-ynyl)oxime hydrochloride;
1-Azabicyclo[2.2.2]octan-3-one, O-(2-pentyn-4-enyl)oxime hydrochloride;
1-Azabicyclo[2.2.2]octan-3-one, O-(2,4-pentadiynyl)oxime hydrochloride; and
1-Azabicyclo[2.2.2]octan-3-one, O-(1-ethynylpropynyl)oxime hydrochloride.

9. A process according to Claim 1 for the preparation of compounds of Claim 1 which is 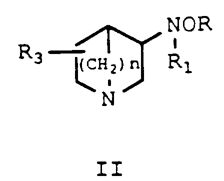 wherein n, R₃, R₁, and R have the meanings defined in Claim 1.

10. A process according to Claim 1 for the preparation of compounds of Claim 1 having the formula: 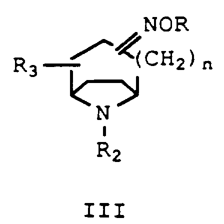 wherein n, R, R₂, and R₃ have the meanings defined in Claim 1.

11. A process according to Claim 1 for the preparation of compounds of Claim 10 wherein n is one.

12. A process according to Claim 1 for the preparation of compounds of Claim 11 which is N-methyl-8-azabicyclo[3.2.1]octa-3-one methyl oxime hydrochloride or N-methyl-8-azabicyclo [3.2.1.] octa-2-one methyloxime hydrochloride.

13. A process according to Claim 1 for the preparation of compounds of Claim 1 having the formula: 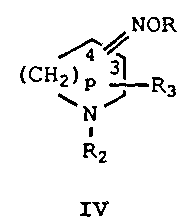 wherein p, R, R₂, and R₃ have the meanings defined in Claim 1.

14. A process according to Claim 1 for the preparation of compounds of Claim 13 wherein p is 2.

15. A process according to Claim 1 for the preparation or compounds of Claim 14 which is 4-piperidinone O-methyloxime hydrochloride; or 1-methyl-4-piperidinone O-methyloxime hydrochloride.

16. A process for the preparation of a pharmaceutical composition comprising the incorporation of an analgesically effective amount of a compound prepared according to Claims 1 to 15 into a pharmaceutically acceptable carrier.

17. Use of a compound prepared according to anyone of Claims 1 to 15 for the preparation of a pharmaceutical composition useful for alleviating pain and for treating the symptoms of senile cognitive decline in a patient.

18. A compound as defined in anyone of Claims 1 to 15.

19. A compound as prepared in anyone of Claims 1 to 15.

## Patentansprüche (Patentansprüche für folgende(n) Vertragsstaat(en): AT, BE, CH, DE, DK, FR, GB, IT, LI, LU, NL, SE)

1. Verbindung der folgenden Formeln I, II, III oder IV oder deren Z- oder E-Form oder ein pharmazeutisch akzeptables Salz derselben: 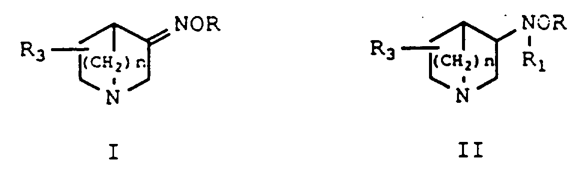 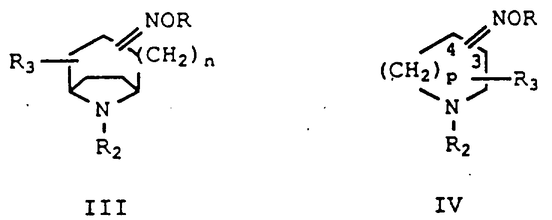 worin bedeuten:
n 1 oder 2;
p 0 oder 1 bis 4;
R₁ und R₂ jeweils Wasserstoff oder gerad- oder verzweigtkettiges Niedrigalkyl mit 1 bis 4 Kohlenstoffatom(en);
R₃ Wasserstoff, eine gerad- oder verzweigtkettige Niedrigalkylgruppe mit 1 bis 6 Kohlenstoffatom(en), Hydroxy, eine gerad- oder verzweigtkettige Niedrigalkoxygruppe mit 1 bis 4 Kohlenstoffatom(en), eine Acyloxygruppe, bei welcher die Acyleinheit 2 bis 5 Kohlenstoffatome aufweist, oder eine Gruppe -(CH₂)_{q}NR₈R₉ mit q gleich 0 oder 1 bis 4 und R₈ und R₉, die gleich oder verschieden sein können, jeweils gleich Wasserstoff oder einer gerad- oder verzweigtkettigen Niedrigalkylgruppe mit 1 bis 4 Kohlenstoffatom(en) oder Cycloalkyl mit 3 bis 6 Kohlenstoffatomen;
R Wasserstoff oder die Gruppe:
-Y-Z
mit Y gleich einer Bindung oder einer gerad- oder verzweigtkettigen Kohlenwasserstoffkette mit 1 bis 20 Kohlenstoffatom(en) und gegebenenfalls 1 bis 4 Doppelbindung(en) und/oder Dreifachbindung(en) und Z gleich Wasserstoff;
eine Cycloalkylgruppe mit 3 bis 6 Kohlenstoffatomen;
eine aromatische Gruppe, ausgewählt aus Phenyl, 2- oder 3-Thienyl, 2- oder 3-Furanyl, 2- oder 3-Pyrrolyl, wobei die aromatische Gruppe unsubstituiert oder ein- oder zweifach durch Chlor, Brom, Fluor, Trifluormethyl, Nitro, Amino, ein- oder zweifach durch gerad- oder verzweigtkettige Niedrigalkylgruppen mit 1 bis 4 Kohlenstoffatom(en) substituiertes Amino oder gerad- oder verzweigtkettiges Alkoxy mit 1 bis 4 Kohlenstoffatom(en) substituiert ist;
-NR₄R₅, worin R₄ und R₅, die gleich oder verschieden sein können, für Wasserstoff oder gerad- oder verzweigtkettiges Alkyl mit 1 bis 4 Kohlenstoffatom(en) stehen; 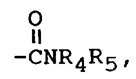
worin R₄ und R₅ die zuvor angegebene Bedeutung besitzen; 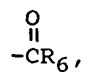
worin R₆ für eine gerad- oder verzweigtkettige Alkylgruppe mit 1 bis 6 Kohlenstoffatom(en) steht;
-CN;
-CO₂R₇, worin R₇ für Wasserstoff, eine gerad- oder verzweigtkettige Niedrigalkylgruppe mit 1 bis 6 Kohlenstoffatom(en) (in gerad- oder verzweigtkettiger Form) oder Benzyl steht; oder
-XR₁₀, worin X für Sauerstoff oder Schwefel steht und R₁₀ eine gerad- oder verzweigtkettige Kohlenwasserstoffkette mit 1 bis 6 Kohlenstoffatom(en) gegebenenfalls mit 1 oder 2 Doppel- oder Dreifachbindung(en) darstellt;
wobei gilt, daß die folgenden Verbindungen ausgenommen sind:
(a) Verbindungen der Formel I mit n = 2 und R gleich Wasserstoff oder mit der Gruppe Y-Z mit Y gleich einer Bindung und Z gleich -C(=O)NR₄R₅ oder -C(=O)R₆ und
(b) Verbindungen der Formeln III und IV mit R gleich Wasserstoff
und deren pharmazeutisch akzeptable Säureadditionssalze.

2. Verbindung nach Anspruch 1 der Formel: 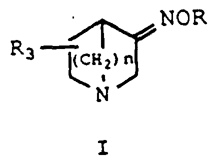 worin n, R₃ und R die in Anspruch 1 angegebene Bedeutung besitzen.

3. Verbindung nach Anspruch 2, worin n = 1 ist.

4. Verbindung nach Anspruch 3, worin R₃ für Wasserstoff steht und R eine von Wasserstoff verschiedene Bedeutung besitzt.

5. Verbindung nach Anspruch 4, nämlich
1-Azabicyclo[2.2.1]heptan-3-on-O-ethyloximhydrochlorid;
1-Azabicyclo[2.2.1]heptan-3-on-O-methyloximhydrochlorid;
1-Azabicyclo[2.2.1]heptan-3-on-O-2-propinyloximhydrochlorid;
1-Azabicyclo[2.2.1]heptan-3-on-O-(phenylmethyl)-oximhydrochlorid;
1-Azabicyclo[2.2.1]heptan-3-on-O-propyloximhydrochlorid;
1-Azabicyclo[2.2.1]heptan-3-on-O-(1-methylethyl)-oximhydrochlorid;
1-Azabicyclo[2.2.1]heptan-3-on-O-(2-propenyl)-oximoxalat;
1-Azabicyclo[2.2.1]heptan-3-on-O-(2-aminoethyl)-oximhydrochlorid;
1-Azabicyclo[2.2.1]heptan-3-on-O-(cyclopropylmethyl)oximhydrochlorid;
1-Azabicyclo[2.2.1]heptan-3-on-O-(2-methoxyethyl)-oximhydrochlorid;
1-Azabicyclo[2.2.1]heptan-3-on-O-phenyloximhydrochlorid;
1-Azabicyclo[2.2.1]heptan-3-on-O-(2-thienylmethyl)-oximhydrochlorid;
1-Azabicyclo[2.2.1]heptan-3-on-O-(2-furylmethyl)oximoxalat;
1-Azabicyclo[2.2.1]heptan-3-on-O-(3-furylmethyl)-oximhydrochlorid;
1-Azabicyclo[2.2.1]heptan-3-on-O-(1-methyl-2-propenyl)-oximoxalat;
1-Azabicyclo[2.2.1]heptan-3-on-O-(3-butenyl)-oximoxalat;
1-Azabicyclo[2.2.1]heptan-3-on-O-(tert.-butyl)-oximhydrochlorid;
1-Azabicyclo[2.2.1]heptan-3-on-O-(2-butinyl)-oximoxalat;
1-Azabicyclo[2.2.1]heptan-3-on-O-(1-methyl-2-butinyl)-oximoxalat;
1-Azabicyclo[2.2.1]heptan-3-on-O-(1-methyl-2-propinyl)-oximoxalat;
1-Azabicyclo[2.2.1]heptan-3-on-O-(E-2-penten-4-inyl)-oximhydrochlorid;
1-Azabicyclo[2.2.1]heptan-3-on-O-(Z-2-penten-4-inyl)-oximhydrochlorid;
1-Azabicyclo[2.2.1]heptan-3-on-O-(2-pentin-4-enyl)-oximhydrochlorid;
1-Azabicyclo[2.2.1]heptan-3-on-O-(2,4-pentadiinyl)-oximhydrochlorid;
1-Azabicyclo[2.2.1]heptan-3-on-O-(1-ethinylpropinyl)-oximhydrochlorid;
[1-Azabicyclo[2.2.1]heptan-3-yliden)-amino]-oxyacetonitrilhydrochlorid;
1-Azabicyclo[2.2.1]heptan-3-on-O-(2,4-pentadienyl)-oximhydrochlorid;
1-Azabicyclo[2.2.1]heptan-3-on-O-(2,4-hexadienyl)-oximhydrochlorid;
1-Azabicyclo[2.2.1]heptan-3-on-O-(Z-2-butenyl)-oximoxalat;
1-Azabicyclo[2.2.1]heptan-3-on-O-(E-2-butenyl)-oximhydrochlorid;
1-Azabicyclo[2.2.1]heptan-3-on-O-(2,5-hexadiinyl)-oximoxalat;
1-Azabicyclo[2.2.1]heptan-3-on-O-(2-penten-4-inyl)-oximoxalat;
1-Azabicyclo[2.2.1]heptan-3-on-O-(4-methoxy-2-butinyl)-oximhydrochlorid;
[[(1-Azabicyclo[2.2.1]heptan-3-yliden)-amino]-oxy]-essigsäuremethylesterhydrochlorid;
1-Azabicyclo[2.2.1]heptan-3-on-O-(2-pentinyl)-oximhydrochlorid;
Z-(+)-1-Azabicyclo[2.2.1]heptan-3-on-O-2-propenyloximoxalat;
E-(±)-1-Azabicyclo[2.2.1]heptan-3-on-O-2-propenyloximoxalat;
Z-(±)-1-Azabicyclo[2.2.1]heptan-3-on-O-2-propinyloximhydrochlorid;
Z-(±)-1-Azabicyclo[2.2.1]heptan-3-on-O-(3-thienylmethyl)-oximoxalat;
E-(±)-1-Azabicyclo[2.2.1]heptan-3-on-O-(3-thienylmethyl)-oximoxalat;
Z-(±)-1-Azabicyclo[2.2.1]heptan-3-on-O-(2-methyl-2-propenyl)-oximoxalat;
E-(±)-1-Azabicyclo[2.2.1]heptan-3-on-O-(2-methyl-2-propenyl)-oximoxalat;
Z-(±)-1-Azabicyclo[2.2.1]heptan-3-on-O-cyclobutyloximhydrochlorid;
E-(±)-1-Azabicyclo[2.2.1]heptan-3-on-O-cyclobutyloximhydrochlorid;
Z-(±)-1-Azabicyclo[2.2.1]heptan-3-on-O-4-pentinyloximoxalat;
E-(±)-1-Azabicyclo[2.2.1]heptan-3-on-O-4-pentinyloximoxalat;
Z-(±)-1-Azabicyclo[2.2.1]heptan-3-on-O-3-pentinyloximoxalat;
E-(±)-1-Azabicyclo[2.2.1]heptan-3-on-O-3-pentinyloximoxalat;
Z-(±)-1-Azabicyclo[2.2.1]heptan-3-on-O-(2,5-heptadiinyl)-oximoxalat;
E-(±)-1-Azabicyclo[2.2.1]heptan-3-on-O-(2,5-heptadiinyl)-oximoxalat;
Z-(±)-1-Azabicyclo[2.2.1]heptan-3-on-O-(2-hexin-5-enyl)-oximoxalat;
Z-(±)-1-Azabicyclo[2.2.1]heptan-3-on-O-(3-phenyl-2-propinyl)-oximoxalat;
E-(±)-1-Azabicyclo[2.2.1]heptan-3-on-O-(3-phenyl-2-propinyl)-oximoxalat;
Z-(±)-1-Azabicyclo[2.2.1]heptan-3-on-O-(4-methyl-2-pentin-4-enyl)-oximoxalat;
E-(±)-1-Azabicyclo[2.2.1]heptan-3-on-O-(4-methylpentin-4-enyl)-oximoxalat;
Z-(±)-1-Azabicyclo[2.2.1]heptan-3-on-O-(2-hexinyl)-oximoxalat;
E-(±)-l-Azabicyclo[2.2.l]heptan-3-on-O-(2-hexinyl)-oximoxalat;
Z-(±)-1-Azabicyclo[2.2.1]heptan-3-on-O-(2-heptinyl)-oximoxalat;
E-(±)-1-Azabicyclo[2.2.1]heptan-3-on-O-(2-heptinyl)-oximoxalat;
Z-(±)-1-Azabicyclo[2.2.1]heptan-3-on-O-(2-(2-propinylthio)-ethyl)-oximoxalat und
E-(±)-1-Azabicyclo[2.2.1]heptan-3-on-O-(2-(2-propinylthio)-ethyl)-oximoxalat.

6. Verbindung nach Anspruch 2, worin n = 2 ist.

7. Verbindung nach Anspruch 6, worin R₃ Wasserstoff bedeutet und R eine von Wasserstoff verschiedene Bedeutung besitzt.

8. Verbindung nach Anspruch 7, nämlich
1-Azabicyclo[2.2.2]octan-3-on-O-ethyloximhydrochlorid;
1-Azabicyclo[2.2.2]octan-3-on-O-methyloxim;
1-Azabicyclo[2.2.2]octan-3-on-O-2-(propinyl)-oxim;
1-Azabicyclo[2.2.2]octan-3-on-O-(phenylmethyl)-oximhydrochlorid;
1-Azabicyclo[2.2.2]octan-3-on-O-propyloximhydrochlorid;
1-Azabicyclo[2.2.2]octan-3-on-O-(1-methylethyl)-oximhydrochlorid;
1-Azabicyclo[2.2.2]octan-3-on-O-(2-propenyl)-oximoxalat;
1-Azabicyclo[2.2.2]octan-3-on-O-(2-aminoethyl)-oximhydrochlorid;
1-Azabicyclo[2.2.2]octan-3-on-O-(cyclopropylmethyl)-oximhydrochlorid;
1-Azabicyclo[2.2.2]octan-3-on-O-(2-methoxyethyl)-oximhydrochlorid;
1-Azabicyclo[2.2.2]octan-3-on-O-phenyloximhydrochlorid;
1-Azabicyclo[2.2.2]octan-3-on-O-(2-thienylmethyl)-oximhydrochlorid;
1-Azabicyclo[2.2.2]octan-3-on-O-(2-furylmethyl)-oximoxalat;
1-Azabicyclo[2.2.2]octan-3-on-O-(3-furylmethyl)-oximhydrochlorid;
1-Azabicyclo[2.2.2]octan-3-on-O-(1-methyl-2-propenyl)-oximoxalat;
1-Azabicyclo[2.2.2]octan-3-on-O-(3-butenyl)-oximoxalat;
1-Azabicyclo[2.2.2]octan-3-on-O-(tert.-butyl)-oximhydrochlorid;
1-Azabicyclo[2.2.2]octan-3-on-O-(2-butinyl)-oximoxalat;
1-Azabicyclo[2.2.2]octan-3-on-O-(1-methyl-2-propinyl)-oximoxalat;
1-Azabicyclo[2.2.2]octan-3-on-O-(1-methyl-2-butinyl)-oximoxalat;
1-Azabicyclo[2.2.2]octan-3-on-O-(E-2-penten-4-inyl)-oximhydrochlorid;
1-Azabicyclo[2.2.2]octan-3-on-O-(Z-2-penten-4-inyl)-oximhydrochlorid;
1-Azabicyclo[2.2.2]octan-3-on-O-(2-pentin-4-enyl)-oximhydrochlorid;
1-Azabicyclo[2.2.2]octan-3-on-O-(2,4-pentadiinyl)-oximhydrochlorid und
1-Azabicyclo[2.2.2]octan-3-on-O-(1-ethinylpropinyl)-oximhydrochlorid.

9. Verbindung nach Anspruch 1, nämlich 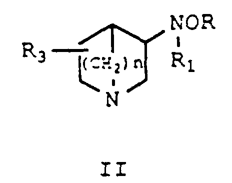 worin n, R₃, R₁ und R die in Anspruch 1 angegebene Bedeutung besitzen.

10. Verbindung nach Anspruch 1 mit der Formel: 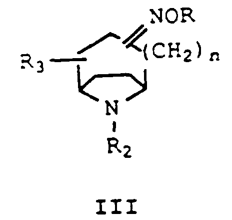 worin n, R, R₂ und R₃ die in Anspruch 1 angegebene Bedeutung besitzen.

11. Verbindung nach Anspruch 10, worin n = 1 ist.

12. Verbindung nach Anspruch 11, nämlich N-Methyl-8-azabicyclo[3.2.1]octa-3-on-methyloximhydrochlorid oder N-Methyl-8-azabicyclo[3.2.1]octa-2-on-methyloximhydrochlorid.

13. Verbindung nach Anspruch 1 mit der Formel: 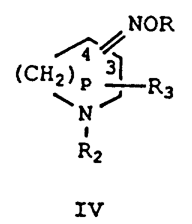 worin p, R, R₂ und R₃ die in Anspruch 1 angegebene Bedeutung besitzen.

14. Verbindung nach Anspruch 13, worin p = 2 ist.

15. Verbindung nach Anspruch 14, nämlich 4-Piperidinon-O-methyloximhydrochlorid oder 1-Methyl-4-piperidinon-O-methyloximhydrochlorid.

16. Arzneimittelzubereitung, umfassend eine Verbindung nach Ansprüchen 1 bis 15 zusammen mit einem pharmazeutisch akzeptablen Träger.

17. Verwendung einer Verbindung nach Ansprüchen 1 bis 15 zur Herstellung einer Arzneimittelzubereitung zur Schmerzlinderung oder Behandlung der Symptome seniler Wahrnehmungsschwäche bei einem Patienten.

18. Verfahren zur Herstellung einer Verbindung nach Ansprüchen 1 bis 15, umfassend die Herstellung einer Verbindung der Formeln I, III oder IV durch Umsetzen eines Ketons der Formeln: 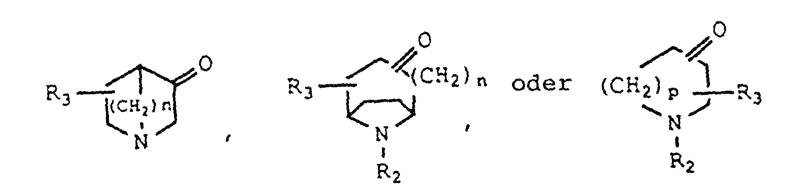 mit einem Aminhydrochlorid der Formel NH₂OR·HCl, worin n, p, R₃, R₂ und R die in Anspruch 1 angegebene Bedeutung besitzen, in Methanol bei Raumtemperatur, und die Gewinnung von Verbindungen der Formel II durch Reduktion von Verbindungen der Formel I und, gewünschtenfalls, Ausbilden eines pharmazeutisch akzeptablen Salzes mit einer organischen oder anorganischen Säure.

## Patentansprüche (Patentansprüche für folgende(n) Vertragsstaat(en): ES, GR)

1. Verfahren zur Herstellung einer Verbindung der folgenden Formeln I, II, III oder IV oder einer Z- oder E-Form derselben oder eines pharmazeutisch akzeptablen Salzes derselben: 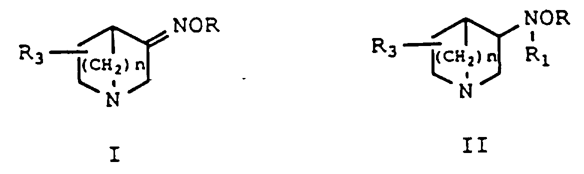 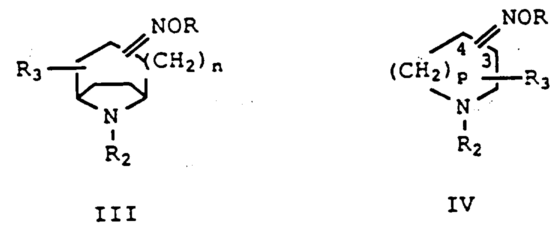 worin bedeuten:
n 1 oder 2;
p 0 oder 1 bis 4;
R₁ und R₂ jeweils Wasserstoff oder gerad- oder verzweigtkettiges Niedrigalkyl mit 1 bis 4 Kohlenstoffatom(en);
R₃ Wasserstoff, eine gerad- oder verzweigtkettige Niedrigalkylgruppe mit 1 bis 6 Kohlenstoffatom(en), Hydroxy, eine gerad- oder verzweigtkettige Niedrigalkoxygruppe mit 1 bis 4 Kohlenstoffatom(en), eine Acyloxygruppe, bei welcher die Acyleinheit 2 bis 5 Kohlenstoffatome aufweist, oder eine Gruppe -(CH₂)_{q}NR₈R₉ mit q gleich 0 oder 1 bis 4 und R₈ und R₉, die gleich oder verschieden sein können, jeweils gleich Wasserstoff oder einer gerad- oder verzweigtkettigen Niedrigalkylgruppe mit 1 bis 4 Kohlenstoffatom(en) oder Cycloalkyl mit 3 bis 6 Kohlenstoffatomen;
R Wasserstoff oder die Gruppe:
-Y-Z
mit Y gleich einer Bindung oder einer gerad- oder verzweigtkettigen Kohlenwasserstoffkette mit 1 bis 20 Kohlenstoffatom(en) und gegebenenfalls 1 bis 4 Doppelbindung(en) und/oder Dreifachbindung(en) und Z gleich Wasserstoff;
eine Cycloalkylgruppe mit 3 bis 6 Kohlenstoffatomen; eine aromatische Gruppe, ausgewählt aus Phenyl, 2- oder 3-Thienyl, 2- oder 3-Furanyl, 2- oder 3-Pyrrolyl, wobei die aromatische Gruppe unsubstituiert oder ein- oder zweifach durch Chlor, Brom, Fluor, Trifluormethyl, Nitro, Amino, ein- oder zweifach durch gerad- oder verzweigtkettige Niedrigalkylgruppen mit 1 bis 4 Kohlenstoffatom(en) substituiertes Amino oder gerad- oder verzweigtkettiges Alkoxy mit 1 bis 4 Kohlenstoffatom(en) substituiert ist;
-NR₄R₅, worin R₄ und R₅, die gleich oder verschieden sein können, für Wasserstoff oder gerad- oder verzweigtkettiges Alkyl mit 1 bis 4 Kohlenstoffatom(en) stehen; 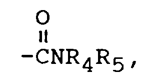
worin R₄ und R₅ die zuvor angegebene Bedeutung besitzen; 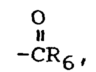
worin R₆ für eine gerad- oder verzweigtkettige Alkylgruppe mit 1 bis 6 Kohlenstoffatom(en) steht;
-CN;
-CO₂R₇, worin R₇ für Wasserstoff, eine gerad- oder verzweigtkettige Niedrigalkylgruppe mit 1 bis 6 Kohlenstoffatom(en) (in gerad- oder verzweigtkettiger Form) oder Benzyl steht; oder
-XR₁₀, worin X für Sauerstoff oder Schwefel steht und R₁₀ eine gerad- oder verzweigtkettige Kohlenwasserstoffkette mit 1 bis 6 Kohlenstoffatom(en) gegebenenfalls mit 1 oder 2 Doppel- oder Dreifachbindung(en) darstellt;
wobei gilt, daß die folgenden Verbindungen ausgenommen sind:
(a) Verbindungen der Formel I mit n = 2 und R gleich Wasserstoff oder mit der Gruppe Y-Z mit Y gleich einer Bindung und Z gleich -C(=O)NR₄R₅ oder -C(=O)R₆ und
(b) Verbindungen der Formeln III und IV mit R gleich Wasserstoff
oder von pharmazeutisch akzeptablen Säureadditionssalzen derselben;
umfassend die Gewinnung einer Verbindung der Formel I, III oder IV durch Umsetzen eines Ketons der Formeln: 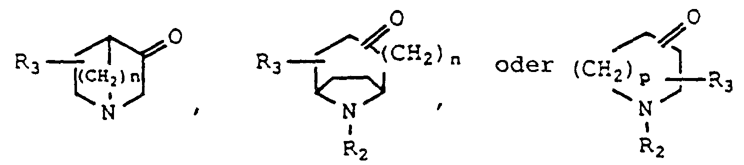 mit einem Aminhydrochlorid der Formel NH₂OR·HCl, worin n, p, R₃, R₂ und R die oben angegebene Bedeutung beitzen, in Methanol bei Raumtemperatur,
oder die Gewinnung von Verbindungen der Formel II durch Reduktion von Verbindungen der Formel I und, gewünschtenfalls, Ausbilden eines pharmazeutisch akzeptablen Salzes mit einer organischen oder anorganischen Säure.

2. Verfahren nach Anspruch 1 zur Herstellung von Verbindungen nach Anspruch 1 der Formel: 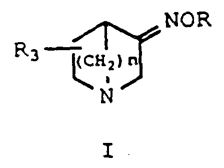 worin n, R₃ und R die in Anspruch 1 angegebene Bedeutung besitzen.

3. Verfahren nach Anspruch 1 zur Herstellung von Verbindungen nach Anspruch 2, worin n = 1 ist.

4. Verfahren nach Anspruch 1 zur Herstellung von Verbindungen nach Anspruch 3, worin R₃ für Wasserstoff steht und R eine von Wasserstoff verschiedene Bedeutung besitzt.

5. Verfahren nach Anspruch 1 zur Herstellung von Verbindungen nach Anspruch 4, nämlich
1-Azabicyclo[2.2.1]heptan-3-on-O-ethyloximhydrochlorid;
1-Azabicyclo[2.2.1]heptan-3-on-O-methyloximhydrochlorid;
1-Azabicyclo[2.2.1]heptan-3-on-O-2-propinyloximhydrochlorid;
1-Azabicyclo[2.2.1]heptan-3-on-O-(phenylmethyl)-oximhydrochlorid;
1-Azabicyclo[2.2.1]heptan-3-on-O-propyloximhydrochlorid;
1-Azabicyclo[2.2.1]heptan-3-on-O-(1-methylethyl)-oximhydrochlorid;
1-Azabicyclo[2.2.1]heptan-3-on-O-(2-propenyl)-oximoxalat;
1-Azabicyclo[2.2.1]heptan-3-on-O-(2-aminoethyl)-oximhydrochlorid;
1-Azabicyclo[2.2.1]heptan-3-on-O-(cyclopropylmethyl)-oximhydrochlorid;
1-Azabicyclo[2.2.1]heptan-3-on-O-(2-methoxyethyl)-oximhydrochlorid;
1-Azabicyclo[2.2.1]heptan-3-on-O-phenyloximhydrochlorid;
1-Azabicyclo[2.2.1]heptan-3-on-O-(2-thienylmethyl)-oximhydrochlorid;
1-Azabicyclo[2.2.1]heptan-3-on-O-(2-furylmethyl)-oximoxalat;
1-Azabicyclo[2.2.1]heptan-3-on-O-(3-furylmethyl)-oximhydrochlorid;
1-Azabicyclo[2.2.1]heptan-3-on-O-(1-methyl-2-propenyl)-oximoxalat;
1-Azabicyclo[2.2.1]heptan-3-on-O-(3-butenyl)-oximoxalat;
1-Azabicyclo[2.2.1]heptan-3-on-O-(tert.-butyl)-oximhydrochlorid;
1-Azabicyclo[2.2.1]heptan-3-on-O-(2-butinyl)-oximoxalat;
1-Azabicyclo[2.2.1]heptan-3-on-O-(1-methyl-2-butinyl)-oximoxalat;
1-Azabicyclo[2.2.1]heptan-3-on-O-(1-methyl-2-propinyl)-oximoxalat;
1-Azabicyclo[2.2.1]heptan-3-on-O-(E-2-penten-4-inyl)-oximhydrochlorid;
1-Azabicyclo[2.2.1]heptan-3-on-O-(Z-2-penten-4-inyl)-oximhydrochlorid;
1-Azabicyclo[2.2.1]heptan-3-on-O-(2-pentin-4-enyl)-oximhydrochlorid;
1-Azabicyclo[2.2.1]heptan-3-on-O-(2,4-pentadiinyl)-oximhydrochlorid;
1-Azabicyclo[2.2.1]heptan-3-on-O-(1-ethinylpropinyl)-oximhydrochlorid;
[1-Azabicyclo[2.2.1]heptan-3-yliden)-amino]-oxyacetonitrilhydrochlorid;
1-Azabicyclo[2.2.1]heptan-3-on-O-(2,4-pentadienyl)-oximhydrochlorid;
1-Azabicyclo[2.2.1]heptan-3-on-O-(2,4-hexadienyl)-oximhydrochlorid;
1-Azabicyclo[2.2.1]heptan-3-on-O-(Z-2-butenyl)-oximoxalat;
1-Azabicyclo[2.2.1]heptan-3-on-O-(E-2-butenyl)-oximhydrochlorid;
1-Azabicyclo[2.2.1]heptan-3-on-O-(2,5-hexadiinyl)-oximoxalat;
1-Azabicyclo[2.2.1]heptan-3-on-O-(2-penten-4-inyl)-oximoxalat;
1-Azabicyclo[2.2.1]heptan-3-on-O-(4-methoxy-2-butinyl)-oximhydrochlorid;
[[(1-Azabicyclo[2.2.1]heptan-3-yliden)-amino]-oxy]-essigsäuremethylesterhydrochlorid;
1-Azabicyclo[2.2.1]heptan-3-on-O-(2-pentinyl)-oximhydrochlorid;
Z-(±)-1-Azabicyclo[2.2.1]heptan-3-on-O-2-propenyloximoxalat;
E-(±)-1-Azabicyclo[2.2.1]heptan-3-on-O-2-propenyloximoxalat;
Z-(±)-1-Azabicyc1o[2.2.1]heptan-3-on-O-2-propinyloximhydrochlorid;
Z-(±)-1-Azabicyclo[2.2.1]heptan-3-on-O-(3-thienylmethyl)-oximoxalat;
E-(±)-1-Azabicyclo[2.2.1]heptan-3-on-O-(3-thienylmethyl)-oximoxalat;
Z-(±)-1-Azabicyclo[2.2.1]heptan-3-on-O-(2-methyl-2-propenyl)-oximoxalat;
E-(±)-1-Azabicyclo[2.2.1]heptan-3-on-O-(2-methyl-2-propenyl)-oximoxalat;
Z-(±)-1-Azabicyclo[2.2.1]heptan-3-on-O-cyclobutyloximhydrochlorid;
E-(±)-1-Azabicyclo[2.2.1]heptan-3-on-O-cyclobutyloximhydrochlorid;
Z-(±)-1-Azabicyclo[2.2.1]heptan-3-on-O-4-pentinyloximoxalat;
E-(±)-1-Azabicyclo[2.2.1]heptan-3-on-O-4-pentinyloximoxalat;
Z-(±)-1-Azabicyclo[2.2.1]heptan-3-on-O-3-pentinyloximoxalat;
E-(±)-1-Azabicyclo[2.2.1]heptan-3-on-O-3-pentinyloximoxalat;
Z-(±)-1-Azabicyclo[2.2.1]heptan-3-on-O-(2,5-heptadiinyl)-oximoxalat;
E-(±)-1-Azabicyclo[2.2.1]heptan-3-on-O-(2,5-heptadiinyl)-oximoxalat;
Z-(±)-1-Azabicyclo[2.2.1]heptan-3-on-O-(2-hexin-5-enyl)-oximoxalat;
Z-(±)-1-Azabicyclo[2.2.1]heptan-3-on-O-(3-phenyl-2-propinyl)-oximoxalat;
E-(±)-1-Azabicyclo[2.2.1]heptan-3-on-O-(3-phenyl-2-propinyl)-oximoxalat;
Z-(±)-1-Azabicyclo[2.2.1]heptan-3-on-O-(4-methyl-2-pentin-4-enyl)-oximoxalat;
E-(±)-1-Azabicyclo[2.2.1]heptan-3-on-O-(4-methylpentin-4-enyl)-oximoxalat;
Z-(±)-1-Azabicyclo[2.2.1]heptan-3-on-O-(2-hexinyl)-oximoxalat;
E-(±)-1-Azabicyclo[2.2.1]heptan-3-on-O-(2-hexinyl)-oximoxalat;
Z-(±)-1-Azabicyclo[2.2.1]heptan-3-on-O-(2-heptinyl)-oximoxalat;
E-(±)-1-Azabicyclo[2.2.1]heptan-3-on-O-(2-heptinyl)-oximoxalat;
Z-(±)-1-Azabicyclo[2.2.l]heptan-3-on-O-(2-(2-propinylthio)-ethyl)-oximoxalat und
E-(±)-1-Azabicyclo[2.2.l]heptan-3-on-O-(2-(2-propinylthio)-ethyl)-oximoxalat.

6. Verfahren nach Anspruch 1 zur Herstellung von Verbindungen nach Anspruch 2, worin n = 2 ist.

7. Verfahren nach Anspruch 1 zur Herstellung von Verbindungen nach Anspruch 6, worin R₃ Wasserstoff bedeutet und R eine von Wasserstoff verschiedene Bedeutung besitzt.

8. Verfahren nach Anspruch 1 zur Herstellung von Verbindungen nach Anspruch 7, nämlich
1-Azabicyclo[2.2.2]octan-3-on-O-ethyloximhydrochlorid;
1-Azabicyclo[2.2.2]octan-3-on-O-methyloxim;
1-Azabicyclo[2.2.2]octan-3-on-O-2-(propinyl)-oxim;
1-Azabicyclo[2.2.2]octan-3-on-O-(phenylmethyl)-oximhydrochlorid;
1-Azabicyclo[2.2.2]octan-3-on-O-propyloximhydrochlorid;
1-Azabicyclo[2.2.2]octan-3-on-O-(1-methylethyl)-oximhydrochlorid;
1-Azabicyclo[2.2.2]octan-3-on-O-(2-propenyl)-oximoxalat;
1-Azabicyclo[2.2.2]octan-3-on-O-(2-aminoethyl)-oximhydrochlorid;
1-Azabicyclo[2.2.2]octan-3-on-O-(cyclopropylmethyl)-oximhydrochlorid;
1-Azabicyclo[2.2.2]octan-3-on-O-(2-methoxyethyl)-oximhydrochlorid;
1-Azabicyclo[2.2.2]octan-3-on-O-phenyloximhydrochlorid;
1-Azabicyclo[2.2.2]octan-3-on-O-(2-thienylmethyl)-oximhydrochlorid;
1-Azabicyclo[2.2.2]octan-3-on-O-(2-furylmethyl)-oximoxalat;
1-Azabicyclo[2.2.2]octan-3-on-O-(3-furylmethyl)-oximhydrochlorid;
1-Azabicyclo[2.2.2]octan-3-on-O-(1-methyl-2-propenyl)-oximoxalat;
1-Azabicyclo[2.2.2]octan-3-on-O-(3-butenyl)-oximoxalat;
1-Azabicyclo[2.2.2]octan-3-on-O-(tert.-butyl)-oximhydrochlorid;
1-Azabicyclo[2.2.2]octan-3-on-O-(2-butinyl)-oximoxalat;
1-Azabicyclo[2.2.2]octan-3-on-O-(1-methyl-2-propinyl)-oximoxalat;
1-Azabicyclo[2.2.2]octan-3-on-O-(1-methyl-2-butinyl)-oximoxalat;
1-Azabicyclo[2.2.2]octan-3-on-O-(E-2-penten-4-inyl)-oximhydrochlorid;
1-Azabicyclo[2.2.2]octan-3-on-O-(Z-2-penten-4-ynyl)-oximhydrochlorid;
1-Azabicyclo[2.2.2]octan-3-on-O-(2-pentin-4-enyl)-oximhydrochlorid;
1-Azabicyclo[2.2.2]octan-3-on-O-(2,4-pentadiinyl)-oximhydrochlorid und
1-Azabicyclo[2.2.2]octan-3-on-O-(1-ethinylpropinyl)-oximhydrochlorid.

9. Verfahren nach Anspruch 1 zur Herstellung von Verbindungen nach Anspruch 1, nämlich solche der Formel: 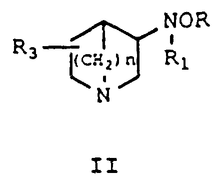 worin n, R₃, R₁ und R die in Anspruch 1 angegebene Bedeutung besitzen.

10. Verfahren nach Anspruch 1 zur Herstellung von Verbindungen nach Anspruch 1 mit der Formel:. 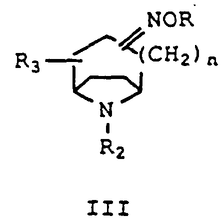 worin n, R, R₂ und R₃ die in Anspruch 1 angegebene Bedeutung besitzen.

11. Verfahren nach Anspruch 1 zur Herstellung von Verbindungen nach Anspruch 10, worin n = 1 ist.

12. Verfahren nach Anspruch 1 zur Herstellung von Verbindungen nach Anspruch 11, nämlich N-Methyl-8-azabicyclo[3.2.1]octa-3-on-methyloximhydrochlorid oder N-Methyl-8-azabicyclo[3.2.1]octa-2-on-methyloximhydrochlorid.

13. Verfahren nach Anspruch 1 zur Herstellung von Verbindungen nach Anspruch 1 der Formel: 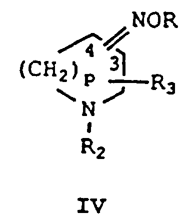 worin p, R, R₂ und R₃ die in Anspruch 1 angegebene Bedeutung besitzen.

14. Verfahren nach Anspruch 1 zur Herstellung von Verbindungen nach Anspruch 13, worin p = 2 ist.

15. Verfahren nach Anspruch 1 zur Herstellung von Verbindungen nach Anspruch 14, nämlich 4-Piperidinon-O-methyloximhydrochlorid oder l-Methyl-4-piperidinon-O-methyloximhydrochlorid.

16. Verfahren zur Herstellung einer Arzneimittelzubereitung, umfassend das Einarbeiten einer analgetisch wirksamen Menge einer nach Ansprüchen 1 bis 15 hergestellten Verbindung in einen pharmazeutisch akzeptablen Träger.

17. Verwendung einer nach einem der Ansprüche 1 bis 15 hergestellten Verbindung zur Bereitstellung eine pharmazeutischen Zubereitung zur Schmerzlinderung und Behandlung der Symptome seniler Wahrnehmungsschwäche bei einem Patienten.

18. Verbindung entsprechend der Definition nach einem der Ansprüche 1 bis 15.

19. Verbindung, hergestellt gemäß einem der Ansprüche 1 bis 15.

## Revendications (Revendications pour l'(les) Etat(s) contractant(s) suivant(s): AT, BE, CH, DE, DK, FR, GB, IT, LI, LU, NL, SE)

1. Un composé de formule générale I, II, III ou IV, ou leurs formes Z ou E, ou un sel pharmaceutiquement acceptable en dérivant: 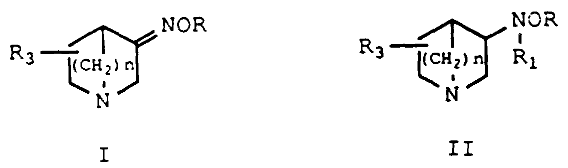 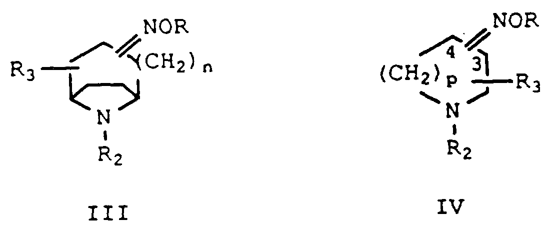 dans lesquelles:
n est égal à 1 ou 2;
p est égal à 0 ou représente un nombre de 1 à 4;
chaque radical R₁ et R₂ représente un atome d'hydrogène ou un radical alkyle inférieur linéaire ou ramifié comportant de 1 à 4 atomes de carbone;
R₃ représente un atome d'hydrogène, un radical alkyle inférieur, linéaire ou ramifié, comportant de 1 à 6 atomes de carbone, un groupe hydroxy, un radical alcoxy inférieur, linéaire ou ramifié, comportant de 1 à 4 atomes de carbone, un groupe acyloxy dans lequel la partie acyle comporte de 2 à 5 atomes de carbone, ou le groupe -(CH₂)_{q}NR₈R₉ dans lequel q est égal à 0 ou représente un nombre de 1 à 4 et chaque radical R₈ et R₉ est identique ou différent et représente un atome d'hydrogène ou un radical alkyle inférieur, linéaire ou ramifié comportant de 1 à 4 atomes de carbone; ou cycloalkyle comportant 3 à 6 atomes de carbone;
R représente un atome d'hydrogène ou le groupe:
-Y-Z
dans lequel:
Y est une liaison ou une chaîne hydrocarbonée linéaire ou ramifiée comportant de 1 à 20 atomes de carbone, et comportant éventuellement de 1 à 4 doubles et/ou triples liaisons; et
Z représente un atome d'hydrogène;
- un groupe cycloalkyle comportant de 3 à 6 atomes de carbone;
- un groupe aromatique sélectionné parmi phényle, 2- ou 3-thiényle, 2- ou 3-furanyle, 2- ou 3-pyrrolyle, dans lesquels le groupe aromatique est non substitué ou est substitué par un ou deux substituants sélectionnés parmi atomes de chlore, brome, fluor, groupes trifluorométhyle, nitro, amino, amino substitué par un ou deux radicaux alkyles inférieurs, linéaires ou ramifiés, comportant de 1 à 4 atomes de carbone, ou alcoxy, linéaire ou ramifié, comportant de 1 à 4 atomes de carbone;
-NR₄R₅ dans lequel R₄ et R₅ sont identiques ou différents et représentent un atome d'hydrogène ou un radical alkyle linéaire ou ramifié comportant de 1 à 4 atomes de carbone; 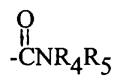
dans lequel R₄ et R₅ sont tels que définis ci-dessus; 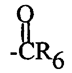
dans lequel R₆ est un radical alkyle linéaire ou ramifié comportant de 1 à 6 atomes de carbone;
-CN;
-CO₂R₇ dans lequel R₇ est un atome d'hydrogène, un radical alkyle inférieur, linéaire ou ramifié, comportant de 1 à 6 atomes de carbone, ou un radical benzyle; ou
-XR₁₀ dans lequel X représente un atome d'hydrogène ou de soufre et R₁₀ représente une chaîne hydrocarbonée linéaire ou ramifiée, comportant de 1 à 6 atomes de carbone, et qui contient éventuellement une ou deux doubles ou triples liaisons;
à condition que les composés suivants soient exclus:
(a) les composés de formule I dans lesquels n est égal à 2, et R représente un atome d'hydrogène, ou le groupe Y-Z dans lequel Y est une liaison et Z est -C(=O)NR₄R₅ ou -C(=O)R₆; et
(b) les composés des formules III et IV, dans lesquelles R est un atome d'hydrogène,
et les sels d'addition d'acide pharmaceutiquement acceptables en dérivant.

2. Un composé selon la revendication 1, représenté par la formule: 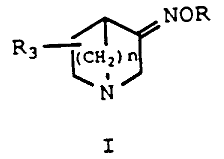 dans laquelle n, R₃ et R sont tels que définis dans la revendication 1.

3. Un composé selon la revendication 2, dans lequel n est égal à un.

4. Un composé selon la revendication 2, dans lequel R₃ représente un atome d'hydrogène et R n'est pas un atome d'hydrogène.

5. Un composé selon la revendication 4, qui correspond à:
- chlorhydrate de 1-azabicyclo[2,2,1]heptan-3-one, O-éthyloxime;
- chlorhydrate de 1-azabicyclo[2,2,1]heptan-3-one, O-méthyloxime;
- chlorhydrate de 1-azabicyclo[2,2,1]heptan-3-one, O-2-propynyloxime;
- chlorhydrate de 1-azabicyclo[2,2,1]heptan-3-one, O-(phénylméthyl)oxime;
- chlorhydrate de 1-azabicyclo[2,2,1]heptan-3-one, O-propyloxime;
- chlorhydrate de 1-azabicyclo[2,2,1]heptan-3-one, O-(1-méthyléthyl)oxime;
- oxalate de 1-azabicyclo[2,2,1]heptan-3-one, O-(2-propényl)oxime;
- chlorhydrate de 1-azabicyclo[2,2,1]heptan-3-one, O-(2-aminoéthyl)oxime;
- chlorhydrate de 1-azabicyclo[2,2,1]heptan-3-one, O-(cyclopropylméthyl) oxime;
- chlorhydrate de 1-azabicyclo[2,2,1]heptan-3-one, O-(2-méthoxyéthyl)oxime;
- chlorhydrate de 1-azabicyclo[2,2,1]heptan-3-one, O-phényloxime;
- chlorhydrate de 1-azabicyclo[2,2,1]heptan-3-one, O-(2-thiénylméthyl)oxime;
- oxalate de 1-azabicyclo[2,2,1]heptan-3-one, O-(2-furylméthyl)oxime;
- chlorhydrate de 1-azabicyclo[2,2,1]heptan-3-one, O-(3-furylméthyl)oxime;
- oxalate de 1-azabicyclo[2,2,1]heptan-3-one, O-(1-méthyl-2-propényl)oxime;
- oxalate de 1-azabicyclo[2,2,1]heptan-3-one, O-(3-butényl)oxime;
- chlorhydrate de 1-azabicyclo[2,2,1]heptan-3-one, O-(tert-butyl)oxime;
- oxalate de 1-azabicyclo[2,2,1]heptan-3-one, O-(2-butynyl)oxime;
- oxalate de 1-azabicyclo[2,2,1]heptan-3-one, O-(1-méthyl-2-butynyl)oxime;
- oxalate de 1-azabicyclo[2,2,1]heptan-3-one, O-(1-méthyl-2-propynyl)oxime;
- chlorhydrate de 1-azabicyclo[2,2,1]heptan-3-one, O-(E-2-pentène-4-ynyl)oxime;
- chlorhydrate de 1-azabicyclo[2,2,1]heptan-3-one, O-(Z-2-pentène-4-ynyl)oxime;
- chlorhydrate de 1-azabicyclo[2,2,1]heptan-3-one, O-(2-pentyn-4-ényl)oxime;
- chlorhydrate de 1-azabicyc1o[2,2,1]heptan-3-one, O-(2,4-pentadiynyl)oxime;
- chlorhydrate de 1-azabicyclo[2,2,1]heptan-3-one, O-(1-éthynylpropynyl) oxime;
- chlorhydrate de [(1-azabicyclo[2,2,1]heptan-3-ylidène)amino]oxyacétonitrile;
- chlorhydrate de 1-azabicyclo[2,2,1]heptan-3-one, O-(2,4-pentadiényl)oxime;
- chlorhydrate de 1-azabicyclo[2,2,1]heptan-3-one, O-(2,4-hexadiényl)oxime;
- oxalate de 1-azabicyclo[2,2,1]heptan-3-one, O-(Z-2-butényl)oxime;
- chlorhydrate de 1-azabicyclo[2,2,1]heptan-3-one, O-(E-2-butényl)oxime;
- oxalate de 1-azabicyclo[2,2,1]heptan-3-one, O-(2,5-hexadiynyl)oxime
- oxalate de 1-azabicyclo[2,2,1]heptan-3-one, O-(2-pentène-4-ynyl)oxime;
- chlorhydrate de 1-azabicyclo[2,2,1]heptan-3-one, O-(4-méthoxy-2-butynyl) oxime;
- chlorhydrate de méthylester d'acide [[(1-azabicyclo[2,2,1]heptan-3-ylidène) amino]oxy]acétique;
- chlorhydrate de 1-azabicyclo[2,2,1]heptan-3-one, O-(2-pentynyl)oxime;
- oxalate de Z-(±)-1-azabicyclo[2,2,1]heptan-3-one, O-2-propényloxime;
- oxalate de E-(±)-1-azabicyclo[2,2,1]heptan-3-one, O-2-propényloxime;
- chlorhydrate de Z-(±)-1-azabicyclo-[2,2,1]heptan-3-one, O-2-propynyloxime;
- oxalate de Z-(±)-1-azabicyclo-[2,2,1]heptan-3-one, O-(3-thiénylméthyl)oxime;
- oxalate de E-(±)-1-azabicyclo-[2,2,1]heptan-3-one, O-(3-thiénylméthyl)oxime;
- oxalate de Z-(±)-1-azabicyclo-[2,2,1]heptan-3-one, O-(2-méthyl-2-propényl) oxime;
- oxalate de E-(±)-1-azabicyclo-[2,2,1]heptan-3-one, O-(2-méthyl-2-propényl) oxime;
- chlorhydrate de Z-(±)-1-azabicyclo-[2,2,1]heptan-3-one, O-cyclobutyloxime;
- chlorhydrate de E-(±)-1-azabicyclo-[2,2,1]heptan-3-one, O-cyclobutyloxime;
- oxalate de Z-(±)-1-azabicyclo-[2,2,1]heptan-3-one, O-4-pentynyloxime;
- oxalate de E-(±)-1-azabicyclo-[2,2,1]heptan-3-one, O-4-pentynyloxime;
- oxalate de Z-(±)-1-azabicyclo-[2,2,1]heptan-3-one, O-3-pentynyloxime;
- oxalate de E-(±)-1-azabicyclo-[2,2,1]heptan-3-one, O-3-pentynyloxime;
- oxalate de Z-(±)-1-azabicyclo-[2,2,1]heptan-3-one, O-(2,5-heptadiynyl)oxime;
- oxalate de E-(±)-1-azabicyclo-[2,2,1]heptan-3-one, O-(2,5-heptadiynyl)oxime;
- oxalate de Z-(±)-1-azabicyclo-[2,2,1]heptan-3-one, O-(2-hexyn-5-ényl)oxime;
- oxalate de Z-(±)-1-azabicyclo-[2,2,1]heptan-3-one, O-(3-phényl-2-propynyl) oxime;
- oxalate de E-(±)-1-azabicyclo-[2,2,1]heptan-3-one, O-(3-phényl-2-propynyl) oxime;
- oxalate de Z-(±)-1-azabicyclo-[2,2,1]heptan-3-one, O-(4-méthyl-2-pentyne-4-ényl)oxime;
- oxalate de E-(±)-1-azabicyclo-[2,2,1]heptan-3-one, O-(4-méthyl-pentyne-4-ényl)oxime;
- oxalate de Z-(±)-1-azabicyclo-[2,2,1]heptan-3-one, O-(2-hexynyl)oxime;
- oxalate de E-(±)-1-azabicyclo-[2,2,1]heptan-3-one, O-(2-hexynyl)oxime;
- oxalate de Z-(±)-1-azabicyclo-[2,2,1]heptan-3-one, O-(2-heptynyl)oxime;
- oxalate de E-(±)-1-azabicyclo-[2,2,1]heptan-3-one, O-(2-heptynyl)oxime;
- oxalate de Z-(±)-1-azabicyclo-[2,2,1]heptan-3-one, O-(2-(2-propynylthio) éthyl)oxime; et
- oxalate de E-(±)-1-azabicyclo-[2,2,1]heptan-3-one, O-(2-(2-propynylthio) éthyl)oxime.

6. Un composé selon la revendication 2, dans lequel n est égal à 2.

7. Un composé selon la revendication 6, dans lequel R₃ est un atome d'hydrogène et R n'est pas un atome d'hydrogène.

8. Un composé selon la revendication 7, qui correspond à:
- chlorhydrate de 1-azabicyclo-[2,2,2]octan-3-one, O-éthyloxime;
- 1-azabicyclo-[2,2,2]octan-3-one, O-méthyloxime,
- 1-azabicyclo-[2,2,2]octan-3-one, O-2-(propynyl)oxime;
- chlorhydrate de 1-azabicyclo-[2,2,2]octan-3-one, O-(phénylméthyl)oxime;
- chlorhydrate de 1-azabicyclo-[2,2,2]octan-3-one, O-propyloxime;
- chlorhydrate de 1-azabicyclo-[2,2,2]octan-3-one, O-(1-méthyléthyl)oxime;
- oxalate de 1-azabicyclo-[2,2,2]octan-3-one, O-(2-propényl)oxime;
- chlorhydrate de 1-azabicyclo-[2,2,2]octan-3-one, O-(2-aminoéthyl)oxime;
- chlorhydrate de 1-azabicyclo-[2,2,2]octan-3-one, O-(cyclopropylméthyl) oxime;
- chlorhydrate de 1-azabicyclo-[2,2,2]octan-3-one, O-(2-méthoxyéthyl)oxime;
- chlorhydrate de 1-azabicyclo-[2,2,2]octan-3-one, O-phényloxime;
- chlorhydrate de 1-azabicyclo-[2,2,2]octan-3-one, O-(2-thiénylméthyl)oxime;
- oxalate de 1-azabicyclo-[2,2,2]octan-3-one, O-(2-furylméthyl)oxime;
- chlorhydrate de 1-azabicyclo-[2,2,2]octan-3-one, O-(3-furylméthyl)oxime;
- oxalate de 1-azabicyclo-[2,2,2]octan-3-one, O-(1-méthyl-2-propényl)oxime;
- oxalate de 1-azabicyclo-[2,2,2]octan-3-one, O-(3-butényl)oxime;
- chlorhydrate de 1-azabicyclo-[2,2,2]octan-3-one, O-(tert-butyl)oxime;
- oxalate de 1-azabicyclo-[2,2,2]octan-3-one, O-(2-butynyl)oxime;
- oxalate de 1-azabicyclo-[2,2,2]octan-3-one, O-(1-méthyl-2-propynyl)oxime;
- oxalate de 1-azabicyclo-[2,2,2]octan-3-one, O-(1-méthyl-2-butynyl)oxime;
- chlorhydrate de 1-azabicyclo-[2,2,2]octan-3-one, O-(E-2-pentène-4-ynyl)-oxime;
- chlorhydrate de 1-azabicyclo-[2,2,2]octan-3-one, O-(Z-2-pentène-4-ynyl)-oxime;
- chlorhydrate de 1-azabicyclo-[2,2,2]octan-3-one, O-(2-pentyne-4-ényl)oxime;
- chlorhydrate de 1-azabicyclo-[2,2,2]octan-3-one, O-(2,4-pentadiynyl)oxime;
et
- chlorhydrate de 1-azabicyclo-[2,2,2]octan-3-one, O-(1-éthynylpropnyl)oxime.

9. Un composé selon la revendication 1, qui est représenté par la formule: 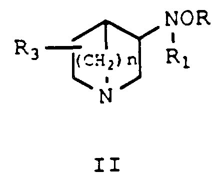 dans laquelle n, R₃, R₁ et R sont tels que définis dans la revendication 1.

10. Un composé selon la revendication 1, représenté par la formule: 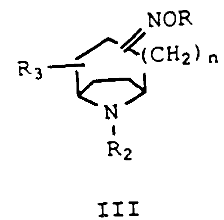 dans laquelle n, R, R₂ et R₃ sont tels que définis dans la revendication 1.

11. Un composé selon la revendication 10, dans lequel n est égal à un.

12. Un composé selon la revendication 11, qui est le chlorhydrate de N-méthyl-8-azabicyclo[3,2,1]octan-3-one méthyloxime ou le chlorhydrate de N-méthyl-8-azabicyclo[3,2,1]octan-2-one méthyloxime.

13. Un composé selon la revendication 1, représenté par la formule: 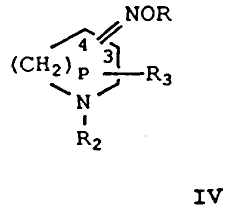 dans laquelle p, R, R₂ et R₃ sont tels que définis dans la revendication 1.

14. Un composé selon la revendication 13, dans lequel p est égal à 2.

15. Un composé selon la revendication 14, qui est le chlorhydrate de 4-piperidinone-O-méthyloxime; ou le chlorhydrate de 1-méthyl-4-pipéridinone-O-méthyloxime.

16. Une composition pharmaceutique comprenant un composé selon les revendications 1 à 15, ainsi qu'un support pharmaceutiquement acceptable.

17. Utilisation d'un composé selon les revendications 1 à 15, pour la préparation d'une composition pharmaceutique pour soulager la douleur ou traiter les symptômes du déclin cognitif sénile chez un patient.

18. Un procédé de préparation d'un composé selon la revendication 1 à 15, qui comprend, pour l'obtention d'un composé de formule I, III ou IV, respectivement, la réaction d'une cétone sélectionnée parmi celles qui répondent aux formules: 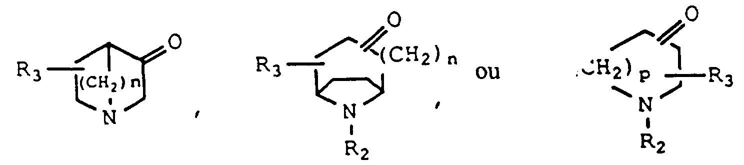 avec un chlorhydrate d'amine représenté par la formule NH₂OR,HCl dans lesquelles n, p, R₃, R₂ et R sont tels que définis dans la revendication 1, dans du méthanol à la température ambiante, et pour obtenir les composés de formule II, la réduction des composés de formule I et, si cela est souhaité, la formation d'un sel pharmaceutiquement acceptable avec un acide organique ou inorganique.

## Revendications (Revendications pour l'(les) Etat(s) contractant(s) suivant(s): ES, GR)

1. Un procédé de préparation d'un composé représenté par les formules suivantes I, II, III ou IV, de leurs formes Z ou E, ou d'un sel pharmaceutiquement acceptable en dérivant: 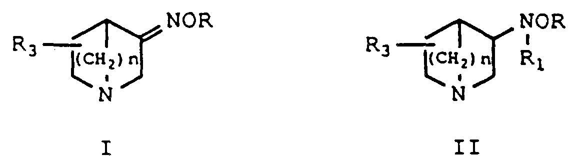 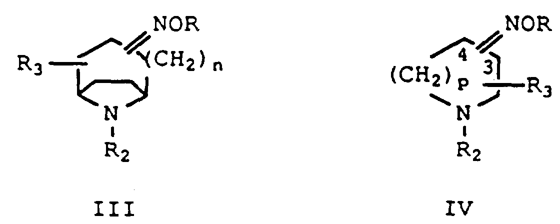 dans lesquelles:
n est égal à 1 ou 2;
p est égal à 0 ou représente un nombre de 1 à 4;
chaque radical R₁ et R₂ représente un atome d'hydrogène ou un radical alkyle inférieur linéaire ou ramifié comportant de 1 à 4 atomes de carbone;
R₃ représente un atome d'hydrogène, un radical alkyle inférieur, linéaire ou ramifié, comportant de 1 à 6 atomes de carbone, un groupe hydroxy, un radical alcoxy inférieur, linéaire ou ramifié, comportant de 1 à 4 atomes de carbone, un groupe acyloxy dans lequel la partie acyle comporte de 2 à 5 atomes de carbone, ou le groupe -(CH₂)_{q}NR₈R₉ dans lequel q est égal à 0 ou représente un nombre de 1 à 4 et chaque radical R₈ et R₉ est identique ou différent et représente un atome d'hydrogène ou un radical alkyle inférieur, linéaire ou ramifié comportant de 1 à 4 atomes de carbone; ou cycloalkyle comportant 3 à 6 atomes de carbone;
R représente un atome d'hydrogène ou le groupe:
-Y-Z
dans lequel:
Y est une liaison ou une chaîne hydrocarbonée linéaire ou ramifiée comportant de 1 à 20 atomes de carbone, et comportant éventuellement de 1 à 4 doubles et/ou triples liaisons; et
Z représente un atome d'hydrogène;
- un groupe cycloalkyle comportant de 3 à 6 atomes de carbone;
- un groupe aromatique sélectionné parmi phényle, 2- ou 3-thiényle, 2- ou 3-furanyle, 2- ou 3-pyrrolyle, dans lesquels le groupe aromatique est non substitué ou est substitué par un ou deux substituants sélectionnés parmi atomes de chlore, brome, fluor, groupes trifluorométhyle, nitro, amino, amino substitué par un ou deux radicaux alkyles inférieurs, linéaires ou ramifiés, comportant de 1 à 4 atomes de carbone, ou alcoxy, linéaire ou ramifié, comportant de 1 à 4 atomes de carbone;
-NR₄R₅ dans lequel R₄ et R₅ sont identiques ou différents et représentent un atome d'hydrogène ou un radical alkyle linéaire ou ramifié comportant de 1 à 4 atomes de carbone; 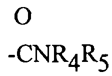
dans lequel R₄ et R₅ sont tels que définis ci-dessus; 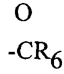
dans lequel R₆ est un radical alkyle linéaire ou ramifié comportant de 1 à 6 atomes de carbone;
-CN;,
-CO₂R₇ dans lequel R₇ est un atome d'hydrogène, un radical alkyle inférieur, linéaire ou ramifié, comportant de 1 à 6 atomes de carbone, ou un radical benzyle; ou
-XR₁₀ dans lequel X représente un atome d'hydrogène ou de soufre et R₁₀ représente une chaîne hydrocarbonée linéaire ou ramifiée, comportant de 1 à 6 atomes de carbone, et qui contient éventuellement une ou deux doubles ou triples liaisons;
à condition que les composés suivants soient exclus:
(a) les composés de formule I dans lesquels n est égal à 2, et R représente un atome d'hydrogène, ou le groupe Y-Z dans lequel Y est une liaison et Z est -C(=O)NR₄R₅ ou -C(=O)R₆; et
(b) les composés des formules III et IV, dans lesquelles R est un atome d'hydrogène,
et les sels d'addition d'acide pharmaceutiquement acceptables en dérivant, qui comprend, pour l'obtention d'un composé de formule I, III ou IV, respectivement, la réaction d'une cétone sélectionnée parmi celles qui répondent aux formules: 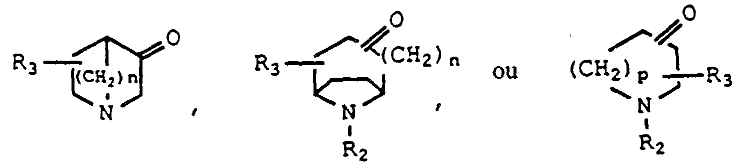 avec un chlorhydrate d'amine représenté par la formule NH₂OR,HCl dans lesquelles n, p, R₃, R₂ et R sont tels que définis dans la revendication 1, dans du méthanol à la température ambiante, et pour obtenir les composés de formule II, la réduction des composés de formule I et, si cela est souhaité, la formation d'un sel pharmaceutiquement acceptable avec un acide organique ou inorganique.

2. Un procédé selon la revendication 1, pour la préparation des composés selon la revendication 1, représenté par la formule: 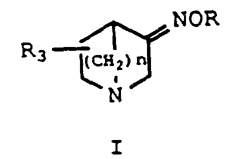 dans laquelle n, R₃ et R sont tels que définis dans la revendication 1.

3. Un procédé selon la revendication 1, pour la préparation des composés selon la revendication 2, dans lequel n est égal à un.

4. Un procédé selon la revendication 1, pour la préparation des composés selon la revendication 3, dans lequel R₃ représente un atome d'hydrogène et R n'est pas un atome d'hydrogène.

5. Un procédé selon la revendication 1, pour la préparation des composés selon la revendication 4, qui correspond à:
- chlorhydrate de 1-azabicyclo[2,2,1]heptan-3-one, O-éthyloxime;
- chlorhydrate de 1-azabicyclo[2,2,1]heptan-3-one, O-méthyloxime;
- chlorhydrate de 1-azabicyclo[2,2,1]heptan-3-one, O-2-propynyloxime;
- chlorhydrate de 1-azabicyclo[2,2,1]heptan-3-one, O-(phénylméthyl)oxime;
- chlorhydrate de 1-azabicyclo[2,2,1]heptan-3-one, O-propyloxime;
- chlorhydrate de 1-azabicyclo[2,2,1]heptan-3-one, O-(1-méthyléthyl)oxime;
- oxalate de l-azabicyclo[2,2,1]heptan-3-one, O-(2-propényl)oxime;
- chlorhydrate de 1-azabicyclo[2,2,1]heptan-3-one, O-(2-aminoéthyl)oxime;
- chlorhydrate de 1-azabicyclo[2,2,1]heptan-3-one, O-(cyclopropylméthyl) oxime;
- chlorhydrate de 1-azabicydo[2,2,1]heptan-3-one, O-(2-méthoxyéthyl)oxime;
- chlorhydrate de l-azabicyclo[2,2,1]heptan-3-one, O-phényloxime;
- chlorhydrate de 1-azabicyclo[2,2,1]heptan-3-one, O-(2-thiénylméthyl)oxime;
- oxalate de 1-azabicyclo[2,2,1]heptan-3-one, O-(2-furylméthyl)oxime;
- chlorhydrate de 1-azabicyclo[2,2,1]heptan-3-one, O-(3-furylméthyl)oxime;
- oxalate de 1-azabicyclo[2,2,1]heptan-3-one, O-(1-méthyl-2-propényl)oxime;
- oxalate de 1-azabicyclo[2,2,1]heptan-3-one, O-(3-butényl)oxime;
- chlorhydrate de 1-azabicyclo[2,2,1]heptan-3-one, O-(tert-butyl)oxime;
- oxalate de 1-azabicyclo[2,2,1]heptan-3-one, O-(2-butynyl)oxime;
- oxalate de 1-azabicyclo[2,2,1]heptan-3-one, O-(1-méthyl-2-butynyl)oxime;
- oxalate de 1-azabicyclo[2,2,1]heptan-3-one, O-(1-méthyl-2-propynyl)oxime;
- chlorhydrate de 1-azabicyclo[2,2,1]heptan-3-one, O-(E-2-pentène-4-ynyl)oxime;
- chlorhydrate de 1-azabicyclo[2,2,1]heptan-3-one, O-(Z-2-pentène-4-ynyl)oxime;
- chlorhydrate de 1-azabicyclo[2,2,1]heptan-3-one, O-(2-pentyn-4-ényl)oxime;
- chlorhydrate de 1-azabicyclo[2,2,1]heptan-3-one, O-(2,4-pentadiynyl)oxime;
- chlorhydrate de 1-azabicyclo[2,2,1]heptan-3-one, O-(1-éthynylpropynyl) oxime;
- chlorhydrate de [(1-azabicyclo[2,2,1]heptan-3-ylidène)amino]oxyacétonitrile;
- chlorhydrate de 1-azabicyclo[2,2,1]heptan-3-one, O-(2,4-pentadiényl)oxime;
- chlorhydrate de 1-azabicyclo[2,2,1]heptan-3-one, O-(2,4-hexadiényl)oxime;
- oxalate de 1-azabicyclo[2,2,1]heptan-3-one, O-(Z-2-butényl)oxime;
- chlorhydrate de 1-azabicyclo[2,2,1]heptan-3-one, O-(E-2-butényl)oxime;
- oxalate de 1-azabicyclo[2,2,1]heptan-3-one, O-(2,5-hexadiynyl)oxime
- oxalate de 1-azabicyclo[2,2,1]heptan-3-one, O-(2-pentène-4-ynyl)oxime;
- chlorhydrate de 1-azabicyclo[2,2,1]heptan-3-one, O-(4-méthoxy-2-butynyl) oxime;
- chlorhydrate de méthylester d'acide [[(1-azabicyclo[2,2,1]heptan-3-ylidène) amino]oxy]acétique;
- chlorhydrate de 1-azabicyclo[2,2,1]heptan-3-one, O-(2-pentynyl)oxime;
- oxalate de Z-(±)-1-azabicyclo[2,2,1]heptan-3-one, O-2-propényloxime;
- oxalate de E-(±)-1-azabicyclo[2,2,1]heptan-3-one, O-2-propényloxime;
- chlorhydrate de Z-(±)-1-azabicyclo-[2,2,1]heptan-3-one, O-2-propynyloxime;
- oxalate de Z-(±)-1-azabicyclo-[2,2,1]heptan-3-one, O-(3-thiénylméthyl)oxime;
- oxalate de E-(±)-1-azabicyclo-[2,2,1]heptan-3-one, O-(3-thiénylméthyl)oxime;
- oxalate de Z-(±)-1-azabicyclo-[2,2,1]heptan-3-one, O-(2-méthyl-2-propényl) oxime;
- oxalate de E-(±)-1-azabicyclo-[2,2,1]heptan-3-one, O-(2-méthyl-2-propényl) oxime;
- chlorhydrate de Z-(±)-1-azabicyclo-[2,2,1]heptan-3-one, O-cyclobutyloxime;
- chlorhydrate de E-(±)-1-azabicyclo-[2,2,1]heptan-3-one, O-cyclobutyloxime;
- oxalate de Z-(±)-1-azabicyclo-[2,2,1]heptan-3-one, O-4-pentynyloxime;
- oxalate de E-(±)-1-azabicyclo-[2,2,1]heptan-3-one, O-4-pentynyloxime;
- oxalate de Z-(±)-1-azabicyclo-[2,2,1]heptan-3-one, O-3-pentynyloxime;
- oxalate de E-(±)-1-azabicyclo-[2,2,1]heptan-3-one, O-3-pentynyloxime;
- oxalate de Z-(±)-1-azabicyclo-[2,2,1]heptan-3-one, O-(2,5-heptadiynyl)oxime;
- oxalate de E-(±)-1-azabicyclo-[2,2,1]heptan-3-one, O-(2,5-heptadiynyl)oxime;
- oxalate de Z-(±)-1-azabicyclo-[2,2,1]heptan-3-one, O-(2-hexyn-5-ényl)oxime;
- oxalate de Z-(±)-1-azabicyclo-[2,2,1]heptan-3-one, O-(3-phényl-2-propynyl) oxime;
- oxalate de E-(±)-1-azabicyclo-[2,2,1]heptan-3-one, O-(3-phényl-2-propynyl) oxime;
- oxalate de Z-(±)-1-azabicyclo-[2,2,1]heptan-3-one, O-(4-méthyl-2-pentyne-4-ényl)oxime;
- oxalate de E-(±)-1-azabicyclo-[2,2,1]heptan-3-one, O-(4-méthyl-pentyne-4-ényl)oxime;
- oxalate de Z-(±)-1-azabicyclo-[2,2,1]heptan-3-one, O-(2-hexynyl)oxime;
- oxalate de E-(±)-1-azabicyclo-[2,2,1]heptan-3-one, O-(2-hexynyl)oxime;
- oxalate de Z-(±)-1-azabicyclo-[2,2,1]heptan-3-one, O-(2-heptynyl)oxime;
- oxalate de E-(±)-1-azabicyclo-[2,2,1]heptan-3-one, O-(2-heptynyl)oxime;
- oxalate de Z-(±)-1-azabicyclo-[2,2,1]heptan-3-one, O-(2-(2-propynylthio) éthyl)oxime; et
- oxalate de E-(±)-1-azabicyclo-[2,2,1]heptan-3-one, O-(2-(2-propynylthio) éthyl)oxime.

6. Un procédé selon la revendication 1 pour la préparation des composés selon la revendication 2, dans lequel n est égal à 2.

7. Un procédé selon la revendication 1 pour la préparation des composés selon la revendication 6, dans lequel R₃ est un atome d'hydrogène et R n'est pas un atome d'hydrogène.

8. Un procédé selon la revendication 1 pour la préparation des composés selon la revendication 7, qui correspondent à:
- chlorhydrate de 1-azabicyclo-[2,2,2]octan-3-one, O-éthyloxime;
- 1-azabicyclo-[2,2,2]octan-3-one, O-méthyloxime,
- 1-azabicyclo-[2,2,2]octan-3-one, O-2-(propynyl)oxime;
- chlorhydrate de 1-azabicyclo-[2,2,2]octan-3-one, O-(phénylméthyl)oxime;
- chlorhydrate de 1-azabicyclo-[2,2,2]octan-3-one, O-propyloxime;
- chlorhydrate de 1-azabicyclo-[2,2,2]octan-3-one, O-(1-méthyléthyl)oxime;
- oxalate de 1-azabicyclo-[2,2,2]octan-3-one, O-(2-propényl)oxime;
- chlorhydrate de 1-azabicyclo-[2,2,2]octan-3-one, O-(2-aminoéthyl)oxime;
- chlorhydrate de 1-azabicyclo-[2,2,2]octan-3-one, O-(cyclopropylméthyl) oxime;
- chlorhydrate de 1-azabicyclo-[2,2,2]octan-3-one, O-(2-méthoxyéthyl)oxime;
- chlorhydrate de 1-azabicyclo-[2,2,2]octan-3-one, O-phényloxime;
- chlorhydrate de 1-azabicyclo-[2,2,2]octan-3-one, O-(2-thiénylméthyl)oxime;
- oxalate de 1-azabicyclo-[2,2,2]octan-3-one, O-(2-furylméthyl)oxime;
- chlorhydrate de 1-azabicyclo-[2,2,2]octan-3-one, O-(3-furylméthyl)oxime;
- oxalate de 1-azabicyclo-[2,2,2]octan-3-one, O-(1-méthyl-2-propényl)oxime;
- oxalate de 1-azabicyclo-[2,2,2]octan-3-one, O-(3-butényl)oxime;
- chlorhydrate de 1-azabicyclo-[2,2,2]octan-3-one, O-(tert-butyl)oxime;
- oxalate de 1-azabicyclo-[2,2,2]octan-3-one, O-(2-butynyl)oxime;
- oxalate de 1-azabicyclo-[2,2,2]octan-3-one, O-(1-méthyl-2-propynyl)oxime;
- oxalate de 1-azabicyclo-[2,2,2]octan-3-one, O-(1-méthyl-2-butynyl)oxime;
- chlorhydrate de 1-azabicyclo-[2,2,2]octan-3-one, O-(E-2-pentène-4-ynyl)-oxime;
- chlorhydrate de 1-azabicyclo-[2,2,2]octan-3-one, O-(Z-2-pentène-4-ynyl)-oxime;
- chlorhydrate de 1-azabicyclo-[2,2,2]octan-3-one, O-(2-pentyne-4-ényl)oxime;
- chlorhydrate de 1-azabicyclo-[2,2,2]octan-3-one, O-(2,4-pentadiynyl)oxime;
et
- chlorhydrate de 1-azabicyclo-[2,2,2]octan-3-one, O-(1-éthynylpropnyl)oxime.

9. Un procédé selon la revendication 1 pour la préparation des composés selon la revendication 1, représentés par la formule: 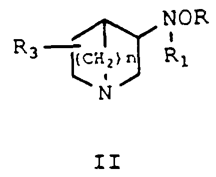 dans laquelle n, R₃, R₁ et R sont tels que définis dans la revendication 1.

10. Un procédé selon la revendication 1 pour la préparation des composés selon la revendication 1, représentés par la formule: 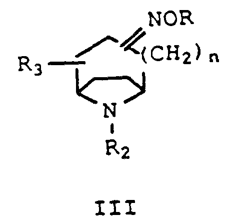 dans laquelle n, R, R₂ et R₃ sont tels que définis dans la revendication 1.

11. Un procédé selon la revendication 1 pour la préparation des composés selon la revendication 10, dans lesquels n est égal à un.

12. Un procédé selon la revendication 1 pour la préparation des composés selon la revendication 11, qui sont le chlorhydrate de N-méthyl-8-azabicyclo [3,2,1]octan-3-one méthyloxime ou le chlorhydrate de N-méthyl-8-azabicyclo[3,2,1] octan-2-one méthyloxime.

13. Un procédé selon la revendication 1 pour la préparation des composés selon la revendication 1, représentés par la formule: 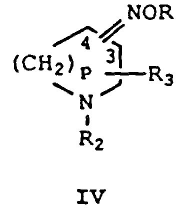 dans laquelle p, R, R₂ et R₃ sont tels que définis dans la revendication 1.

14. Un procédé selon la revendication 1 pour la préparation des composés selon la revendication 13, dans lesquels p est égal à 2.

15. Un procédé selon la revendication 1 pour la préparation des composés selon la revendication 14, qui sont le chlorhydrate de 4-piperidinone-O-méthyloxime ou le chlorhydrate de 1-méthyl-4-pipéridinone-O-méthyloxime.

16. Un procédé de préparation d'une composition pharmaceutique comprenant l'incorporation d'une quantité efficace du point de vue analgésique d'un composé préparé selon les revendications 1 à 15 dans un support pharmaceutiquement acceptable.

17. Utilisation d'un composé préparé selon l'une quelconque des revendications 1 à 15, pour la préparation d'une composition pharmaceutique utile pour soulager la douleur ou pour le traitement des symptômes du déclin cognitif sénile chez un patient.

18. Un composé tel que défini dans l'une quelconque des revendications 1 à 15.

19. Un composé tel que préparé selon l'une quelconque des revendications 1 à 15.
